# EUROPEAN PATENT SPECIFICATION

(11) **EP 1 583 531 B1**
(45) Date of publication and mention of the grant of the patent: **18.04.2007**
(21) Application number: 04701955.9
(22) Date of filing: 14.01.2004
(51) Int. Cl.: A61K 31/437, A61P 1/00, A61P 25/18, A61P 25/22, A61P 25/24, A61P 29/00, C07D 471/04

(54) **HETEROARYL-SUBSTITUTED PYRROLO[2, 3- B]PYRIDINE DERIVATIVES AS CRF RECEPTOR ANTAGONISTS**
HETEROARYL-SUBSTITUIERTE PYRROL[2, 3- B]PYRIDIN-DERIVATE ALS CRF-REZEPTOR-ANTAGONISTEN
DERIVES PYRROLO[2,3- B]PYRIDINE A SUBSTITUTION HETEROARYLE UTILISES COMME ANTAGONISTES DES RECEPTEURS DU CRF

(30) Priority: 16.01.2003 US 440432 P
(43) Date of publication of application: 12.10.2005
(73) Proprietor: SB Pharmco Puerto Rico Inc, Hato Rey, Puerto Rico 00917 (US); NEUROCRINE BIOSCIENCES, INC., San Diego, CA 92121 (US)
(72) Inventor: CASTIGLIONI, Emiliano, I-37100 Verona (IT); DI FABIO, Romano, I-37100 Verona (IT); FERIANI, Aldo, I-37100 Verona (IT); MICHELI, Fabrizio, I-37100 Verona (IT); SABBATINI, Fabio, I-37100 Verona (IT); ST-DENIS, Yves, I-37100 Verona (IT)
(74) Representative: Dolton, Peter Irving Ernest
(86) International application number: PCT/EP2004/000409
(87) International publication number: WO 2004/062665

(56) References cited:
- WO-A-95/33750
- WO-A-98/45295
- WO-A-03/008412

## Description

The present invention relates to bicyclic derivatives, to processes for their preparation, to pharmaceutical compositions containing them and to their use in therapy.

The first corticotropin-releasing factor (CRF) was isolated from ovine hypothalami and identified as a 41-amino acid peptide (Vale et al., Science 213: 1394-1397,1981).
CRF has been found to produce profound alterations in endocrine, nervous and immune system function. CRF is believed to be the major physiological regulator of the basal and stress-release of adrenocorticotropic hormone ("ACTH"), Bendorphin and other proopiomelanocortin ("POMC")-derived peptides from the anterior pituitary (Vale et al., Science 213: 1394-1397,1981).

In addition to its role in stimulating the production of ACTH and POMC, CRF appears to be one of the pivotal central nervous system neurotransmitters and plays a crucial role in integrating the body's overall response to stress.
Administration of CRF directly to the brain elicits behavioral, physiological and endocrine responses identical to those observed for an animal exposed to a stressful environment.
Accordingly, clinical data suggests that CRF receptor antagonists may represent novel antidepressant and/or anxiolytic drugs that may be useful in the treatment of the neuropsychiatric disorders manifesting hypersecretion of CRF.

The first CRF receptor antagonists were peptides (see, e.g., Rivier et al., U.S. Patent No. 4,605,642; Rivier et al., Science 224: 889,1984). While these peptides established that CRF receptor antagonists can attenuate the pharmacological responses to CRF, peptide CRF receptor antagonists suffer from the usual drawbacks of peptide therapeutics including lack of stability and limited oral activity. More recently, small molecule CRF receptor antagonists have been reported.

WO 95/10506 describes inter alia compounds of general formula (A) with general CRF antagonist activity 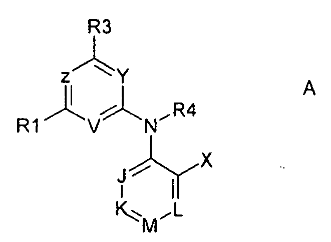 wherein Y may be CR₂₉; V may be nitrogen, Z may be carbon, R₃ may correspond to an amine derivative and R4 may be taken together with R₂₉ to form a 5-membered ring and is -CH(R₂₈) when R₂₉ is-CH(R₃₀). There are no specific disclosures of compounds corresponding to this definition.

WO 95/33750 (and in an analogously way WO01/53263 and EP773023) describes compounds of general formula (B) having CRF antagonistic activity, 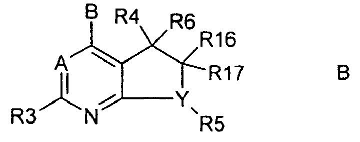 in which A and Y may be nitrogen and carbon and B may correspond to an amine derivative. There are no specific disclosures of compounds corresponding to this definition.

The present invention constitutes a novel selection of the invention object of an International patent application filed by the same Applicant WO 03/008412, describing compounds of the following general formula C: 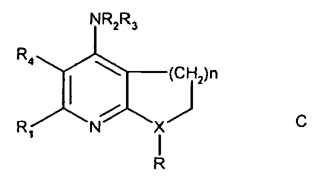 and including in the specification the preparation of the following compounds:
1-(2,4-bis-trifluoromethyl-phenyl)-6-methyl-4-(3-thiazol-2-yl-pyrazol-1-yl)-2,3-dihydro-1*H-*pyrrolo[2,3-*b*]pyridine;
3-methyl-4-[6-methyl-4-(3-thiazol-2-yl-pyrazol-1-yl)-2,3-dihydro-1*H*-pyrrolo[2,3-*b*]pyridin-1-yl]-benzonitrile;
4-[6-methyl-4-(3-thiazol-2-yl-pyrazol-1-yl)-2,3-dihydro-1*H*-pyrrolo[2,3-*b*]pyridin-1-yl]-3-trifluoromethyl-benzonitrile;
6-methyl-1-(2-methyl-4-trifluoromethoxy-phenyl)-4-(3-thiazol-2-yl-pyrazol-1-yl)-2,3-dihydro-1*H*-pyrrolo[2,3-*b*]pyridine;
1-(4-methoxy-2-methyl-phenyl)-6-methyl-4-(3-thiazol-2-yl-pyrazol-1-yl)-2,3-dihydro-1*H-*pyrrolo[2,3-*b*]pyridine
1-(2,4-bis-trifluoromethyl-phenyl)-6-methyl-4-(3-pyridin-2-yl-pyrazol-1-yl)-2,3-dihydro-1*H-*pyrrolo[2,3-*b*]pyridine
4-[1,3']bipyrazolyl-1'-yl-1-(2,4-bis-trifluoromethyl-phenyl)-6-methyl-2,3-dihydro-1*H-*pyrrolo[2,3-*b*]pyridine.

Due to the physiological significance of CRF, the development of biologically-active small molecules having significant CRF receptor binding activity and which are capable of antagonizing the CRF receptor remains a desirable goal. Such CRF receptor antagonists would be useful in the treatment of endocrine, psychiatric and neurologic conditions or illnesses, including stress-related disorders in general.

While significant strides have been made toward achieving CRF regulation through administration of CRF receptor antagonists, there remains a need in the art for effective small molecule CRF receptor antagonists. There is also a need for pharmaceutical compositions containing such CRF receptor antagonists, as well as methods relating to the use thereof to treat, for example, stress-related disorders. The present invention fulfills these needs, and provides other related advantages.

In particular the invention relates to novel compounds which are potent and specific antagonists of corticotropin-releasing factor (CRF) receptors.

The present invention provides compounds of formula (I) including stereoisomers, prodrugs and pharmaceutically acceptable salts or solvates thereof 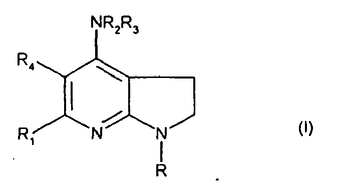 in which:
- R: is pyridine, which may be substituted by 1 to 4 Z groups;
- R₁: is hydrogen, C1-C6 alkyl, C2-C6 alkenyl, C2-C6 alkynyl, halo C1-C6 alkyl, halo C1-C6 alkoxy, halogen, NR₆R₇ or cyano;
- R₂ and R₃: together with N form: 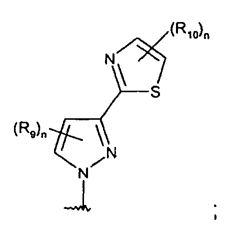
- R₄: is hydrogen;
- R₅: is a C1-C4 alkyl, -OR₆ or -NR₆R₇;
- R₆: is hydrogen or C1-C6 alkyl;
- R₇: is hydrogen or C1-C6 alkyl;
- R₈: is thiazolyl substituted by 1 to 3 R₉ groups;
- R₉: is hydrogen, C3-C7 cycloalkyl, C3-C7 cycloalkenyl, C1-C6 alkyl, C1-C6 alkoxy, halo C1-C6 alkyl, halo C1-C6 alkoxy, hydroxy, halogen, nitro, cyano, -C(O)R₅, C(O)NR₆R₇, phenyl which may be substituted by 1 to 4 R₁₀ groups;
- R₁₀: is C1-C6 alkyl, halo C1-C2 alkyl, halogen, nitro, hydroxy, halo C1-C6 alkoxy, C1-C6 alkoxy,or cyano;
- Z: is selected in a group consisting from halogen, C1-C6 alkyl, C1-C6 alkoxy, halo C1-C6 alkyl, C2-C6 alkenyl, C2-C6 alkynyl, halo C1-C6 alkoxy, -C(O)R₅, -NR₆R₇, cyano, and a group R₈;
- n: is an integer from 1 to 2.

The compounds of the present invention may be in the form of and/or may be administered as a pharmaceutically acceptable salt. For a review on suitable salts see Berge et al, J. Pharm. Sci., 1977, 66, 1-19.

Typically, a pharmaceutical acceptable salt may be readily prepared by using a desired acid or base as appropriate. The salt may precipitate from solution and be collected by filtration or may be recovered by evaporation of the solvent.

Suitable addition salts are formed from acids which form non-toxic salts and examples are hydrochloride, hydrobromide, hydroiodide, sulphate, bisulphate, nitrate, phosphate, hydrogen phosphate, acetate, maleate, malate, fumarate, lactate, tartrate, citrate, formate, gluconate, succinate, piruvate, oxalate, oxaloacetate, trifluoroacetate, saccharate, benzoate, methansulphonate, ethanesulphonate, benzenesulphonate, p-toluensulphonate, methanesulphonic, ethanesulphonic, p-toluenesulphonic, and isethionate.

Pharmaceutically acceptable base salts include ammonium salts, alkali metal salts such as those of sodium and potassium, alkaline earth metal salts such as those of calcium and magnesium and salts with organic bases, including salts of primary, secondary and tertiary amines, such as isopropylamine, diethylamine, ethanolamine, trimethylamine, dicyclohexyl amine and N-methyl-D-glucamine.

Those skilled in the art of organic chemistry will appreciate that many organic compounds can form complexes with solvents in which they are reacted or from which they are precipitated or crystallized. These complexes are known as "solvates". For example, a complex with water is known as a "hydrate". Solvates of the compound of the invention are within the scope of the invention.

In addition, prodrugs are also included within the context of this invention.
As used herein, the term "prodrug" means a compound which is converted within the body, e.g. by hydrolysis in the blood, into its active form that has medical effects. Pharmaceutically acceptable prodrugs are described in T. Higuchi and V. Stella, Prodrugs as Novel Delivery Systems, Vol. 14 of the A.C.S. Symposium Series, Edward B. Roche, ed., Bioreversible Carriers in Drug Design, American Pharmaceutical Association and Pergamon Press, 1987, and in D. Fleisher, S. Ramon and H. Barbra "Improved oral drug delivery: solubility limitations overcome by the use of prodrugs", Advanced Drug Delivery Reviews (1996) 19(2) 115-130, each of which are incorporated herein by reference.

Prodrugs are any covalently bonded carriers that release a compound of structure (I) in vivo when such prodrug is administered to a patient. Prodrugs are generally prepared by modifying functional groups in a way such that the modification is cleaved, either by routine manipulation or in vivo, yielding the parent compound. Prodrugs include, for example, compounds of this invention wherein hydroxy, amine or sulfhydryl groups are bonded to any group that, when administered to a patient, cleaves to form the hydroxy, amine or sulfhydryl groups. Thus, representative examples of prodrugs include (but are not limited to) acetate, formate and benzoate derivatives of alcohol, sulfhydryl and amine functional groups of the compounds of structure (I). Further, in the case of a carboxylic acid (-COOH), esters may be employed, such as methyl esters, ethyl esters, and the like. Esters may be active in their own right and /or be hydrolysable under *in vivo* conditions in the human body. Suitable pharmaceutically acceptable *in vivo* hydrolysable ester groups include those which break down readily in the human body to leave the parent acid or its salt.

With regard to stereoisomers, the compounds of structure (I) may have one or more asymmetric carbon atom and may occur as recemates, racemic mixtures and as individual enantiomers or diastereomers. All such isomeric forms are included within the present invention, including mixtures thereof.

Where a compound of the invention contains an alkenyl or alkenylene group, cis (E) and trans (Z) isomerism may also occur. The present invention includes the individual stereoisomers of the compound of the invention and, where appropriate, the individual tautomeric forms thereof, together with mixtures thereof.

Separation of diastereoisomers or cis and trans isomers may be achieved by conventional techniques, e.g. by fractional crystallisation, chromatography or H.P.L.C. of a stereoisomeric mixture of the agent may also be prepared from a corresponding optically pure intermediate or by resolution, such as H.P.L.C. of the corresponding racemate using a suitable chiral support or by fractional crystallisation of the diastereoisomeric salts formed by reaction of the corresponding racemate with a suitable optically active acid or base, as appropriate.

Furthermore, some of the crystalline forms of the compounds of structure (I) may exist as polymorphs, which are included in the present invention.

The term C1-C6 alkyl as used herein as a group or a part of the group refers to a linear or branched alkyl group containing from 1 to 6 carbon atoms; examples of such groups include methyl, ethyl, propyl, isopropyl, n-butyl, isobutyl, tert butyl, pentyl or hexyl.

The term C3-C7 cycloalkyl group means a saturated hydrocarbon ring of 3 to 7 carbon atom such as, for example, cyclopropyl, cyclobutyl, cyclopentyl, cyclohexyl or cycloheptyl; while unsaturated cycloalkyls include cyclopentenyl and cyclohexenyl, and the like.

The term C3-C7 cycloalkenyl group means a non aromatic monocyclic hydrocarbon ring of 3 to 7 carbon atom, containing from one to two unsaturation such as, cyclopentenyl and cyclohexenyl, and the like.

The term halogen refers to a fluorine, chlorine, bromine or iodine atom.

The term halo C1-C6 alkyl, or halo C1-C2 alkyl means an alkyl group having one or more carbon atoms and wherein at least one hydrogen atom is replaced with halogen such as for example a trifluoromethyl group and the like.

The term C2-C6 alkenyl defines straight or branched chain hydrocarbon radicals containing one or more double bond and having from 2 to 6 carbon atoms such as, for example, ethenyl, 2-propenyl, 3-butenyl, 2-butenyl, 2-pentenyl, 3-pentenyl, 3-methyl-2-butenyl or 3-hexenyl and the like.

The term C1-C6 alkoxy group may be a linear or a branched chain alkoxy group, for example methoxy, ethoxy, propoxy, prop-2-oxy, butoxy, but-2-oxy or methylprop-2-oxy and the like.

The term C1-C6 thioalkyl may be a linear or a branched chain thioalkyl group, for example thiomethyl, thioethyl, thiopropyl, thioisopropyl, thiobutyl, thiosec-butyl, thiotert-butyl and the like.

The term halo C1-C6 alkoxy group may be a C1-C6 alkoxy group as defined before substituted with at least one halogen, preferably fluorine, such as OCHF₂, or OCF₃.

The term C2-C6 alkynyl defines straight or branched chain hydrocarbon radicals containing one or more triple bond and having from 2 to 6 carbon atoms including acetylenyl, propynyl, 1-butynyl, 1-pentynyl, 3-methyl-1-butynyl and the like.

The term aryl means an aromatic carbocyclic moiety such as phenyl, biphenyl or naphthyl.

The term heteroaryl means an aromatic heterocycle ring of 5 to 10 members and having at least one heteroatom selected from nitrogen, oxygen and sulfur, and containing at least 1 carbon atom, including both mono-and bicyclic ring systems.

Representative heteroaryls include (but are not limited to) furyl, benzofuranyl, thiophenyl, benzothiophenyl, pyrrolyl, indolyl, isoindolyl, azaindolyl, pyridyl, quinolinyl, isoquinolinyl, oxazolyl, isooxazolyl, benzoxazolyl, pyrazolyl, imidazolyl, benzimidazolyl, thiazolyl, benzothiazolyl, isothiazolyl, pyridazinyl, pyrimidinyl, pyrazinyl, triazinyl, cinnolinyl, phthalazinyl, triazolyl, tetrazolyl, benzopyrazolyl, and quinazolinyl.

The term 5-6 membered aromatic heterocycle means, a 5-6 monocyclic heterocyclic ring which is aromatic, and which contains from 1 to 4 heteroatoms independently selected from nitrogen, oxygen and sulfur, and wherein the nitrogen and sulfur heteroatoms may be optionally oxidized, and the nitrogen heteroatom may be optionally quaternized. Heterocycles include some of the heteroaryls as defined above. The heterocycle may be attached via any heteroatom or carbon atom. Thus, the term include (but are not limited to) furyl, thiophenyl, pyrrolyl, tetrazolyl, pyrimidinyl, pyridinyl, pyrazinyl, pyrazolyl, thiazolyl, triazolyl, imidazolyl, oxadiazolyl, oxazolyl, and the like. Since such 5-6 membered aromatic heterocycles may be substituted by hydroxy groups, the possible tautomeric forms are also part of the present invention.

Particularly preferred are the compounds of formula (lVa), 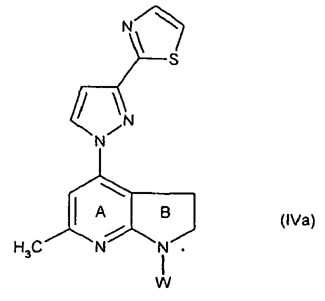 in which W is defined as above.
Specific examples of compounds of general formula (lVa) are included in the Experimental Part.

Preferred compounds according to the invention are:
6-methyl-1-[6-(methyloxy)-2-(trifluoromethyl)-3-pyridinyl]-4-[3-(1,3-thiazol-2-yl)-1*H*-pyrazol-1-yl]-2,3-dihydro-1*H*-pyrrolo [2,3-*b*]pyridine;
6-methyl-1-[2-methyl-6-(methyloxy)-3-pyridinyl]-4-[3-(1,3-thiazol-2-yl)-1*H*-pyrazol-1-yl]-2,3-dihydro-1*H*-pyrrolo [2,3-*b*]pyridine;
1-[2,6-bis(methyloxy)-3-pyridinyl]-6-methyl-4-[3-(1,3-thiazol-2-yl)-1*H*-pyrazol-1-yl]-2,3-dihydro-1*H*-pyrrolo[2,3-*b*]pyridine;
*N,N*,4-trimethyl-5-{6-methyl-4-[3-(1,3-thiazol-2-yl)-1*H*-pyrazol-1-yl]-2,3-dihydro-1*H-*pyrrolo[2,3-*b*]pyridin-1-yl}-2-pyridinamine.

In general, the compounds of structure (I) may be made according to the organic synthesis techniques known to those skilled in this field, as well as by the representative methods set forth in the Examples.

Compounds of formula (I), and salts and solvates thereof, may be prepared by the general methods outlined hereinafter. In the following description, the groups R, R₁, R₂, R₃, R₄, R₅, R₆, R₇, R₈, R₉, R₁₀, Z, W, W₁, W₂, m and n have the meanings as previously defined for compounds of formula (I) unless otherwise stated.

Compounds of formula (I) may be conveniently prepared, starting from compounds of formula (IIIB), according to the following Scheme 1: 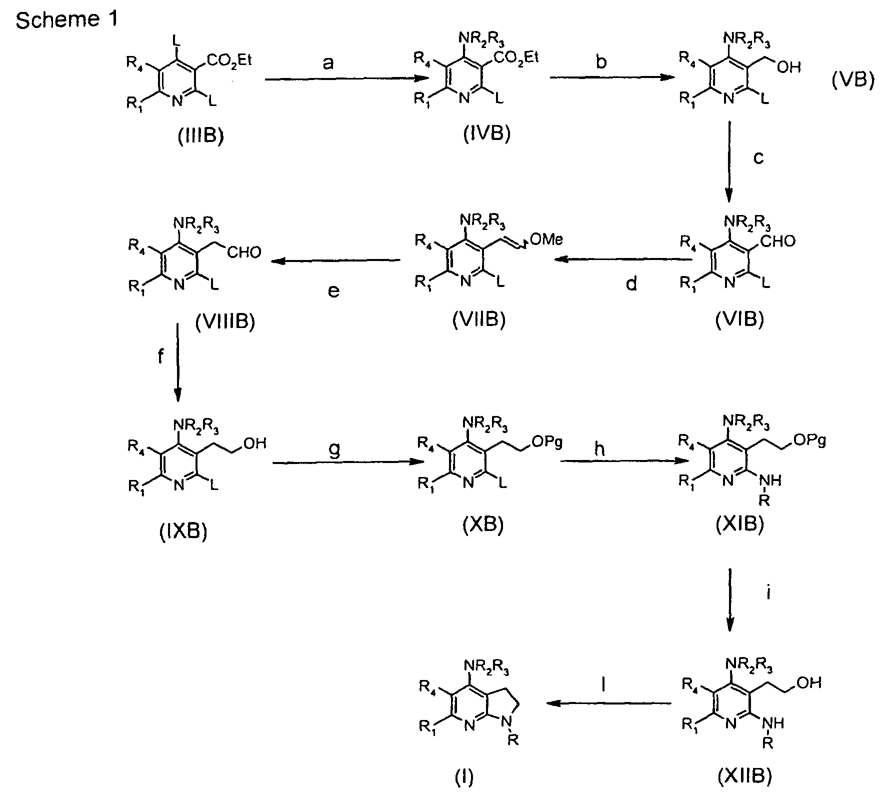 in which
- step a: stands for conversion of the leaving group L, selected in a group consisting from: halogen or reactive residue of sulphonic acid (e.g. mesylate, tosylate), preferably chloride, in the amino group of compounds (IVB), by reaction with the suitable amine NR₂R₃ in basic conditions;
- step b: stands for reduction of the ester group with a suitable reducing agent (such as DIBAI-H) to hydroxy group of compounds (VB);
- step c: stands for oxidation of the hydroxy group with a suitable oxidising agent (such as Dess-Martin periodinane) to aldehyde group of compound (VIB);
- step d: stands for formation of the aldehyde group of compounds (VIIIB) by Wittig reaction in the usual conditions, through formation of enol ether followed by acid hydrolysis (step e);
- step f: stands for reduction of the aldehyde group with a suitable reducing agent (such as NaBH₄) to hydroxy group of compounds (IXB);
- step g: stands for conversion of the hydroxy group in the suitably protected hydroxy group of compounds (XB)(such as TBS: tert-butyldimethylsilyl);
- step h: stands for Buchwald reaction by coupling with the suitable amine RNH₂;
- step i: stands for deprotection reaction to give the hydroxy group of compounds (XIIB);
- step I: stands for intramolecular cyclisation after conversion of the hydroxy group of compounds (XIIB) in a suitable leaving group (such as bromide, by reaction with CBr₄ and PPh₃) to give the final compounds (1).

Alternatively, compounds of formula (I) may be conveniently prepared according to the following Scheme 2: 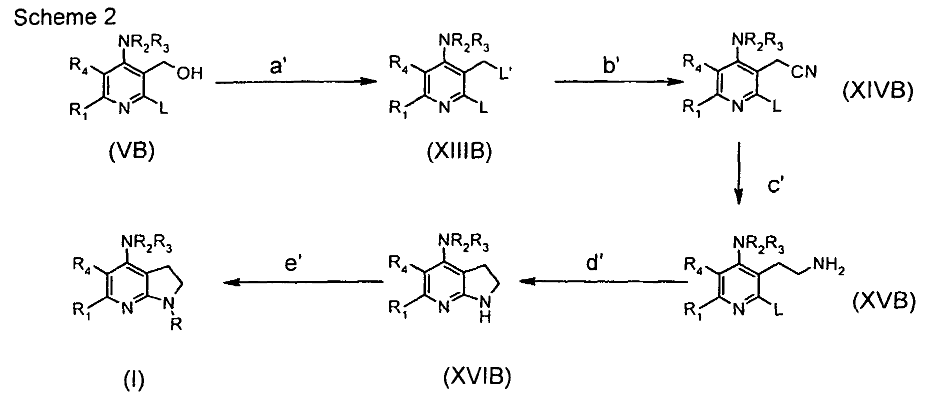 in which
- step a': stands for conversion of the hydroxy group in a suitable leaving group L' of compounds (XIIIB), which, independently from L, has the same definition (e.g mesylate, by reaction with MsCl and Et₃N);
- step b': stands for conversion of L' in the cyano derivative of compounds (XIVB) by reaction with, e.g. KCN in an aprotic dipolar solvent, like DMF;
- step c': stands for reduction of the cyano group with a suitable reducing agent agent (such as BH₃-THF) to the amino group of compound (XVB);
- step d': stands for intramolecular cyclisation of compounds (XVB) by heating in a suitable solvent (such as NMP) at high temperature or intramolecular Copper catalysed cyclisation;
- step e': see step h above. The reaction is done with a suitable aryl halide to give the final compounds (I).

Alternatively, compounds of formula (I) may be conveniently prepared according to the following Scheme 3: 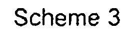 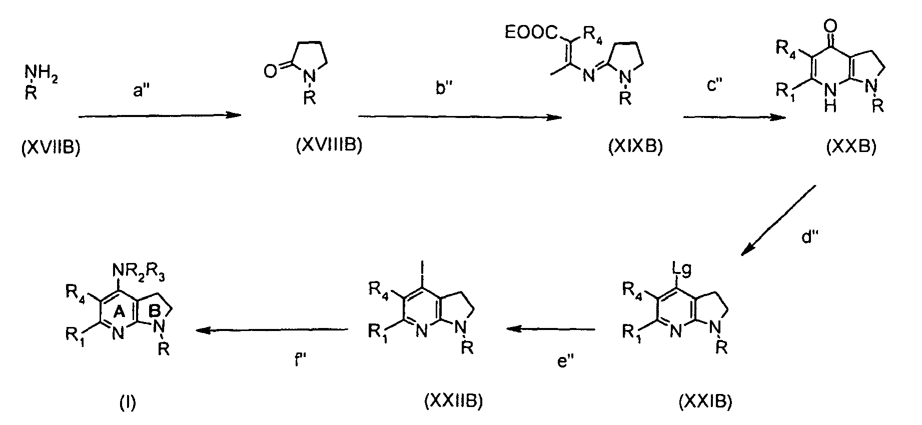 in which:
- step a": stands for the formation of the pyrrolidinone moiety of compounds (XVIIIB), which will form the cycle B present in the final compounds (I), by reacting the compounds (XVIIB) with a reactive derivative of the butyric acid, such as 4-chlorobutyryl chloride; followed by a cyclisation reaction in basic conditions (e.g. KOtBu);
- step b": stands for amidine formation by reacting the compounds (XIXB) with 3-aminocrotonate and POCl₃ ;
- step c": stands for the cyclisation of the compounds (XIXB) in basic conditions (e.g. NaH) to give the pyridinone precursor of cycle A in the final compounds (I);
- step d": stands for the formation of a reactive derivative (i.e. a leaving group, Lg) of the pyridinone (for example selected in a group consisting by triflate, halogen, mesylate) of compounds (XXIB) by reaction with, for example, triflic anhydride;
- step e": stands for nucleophilic displacement of the leaving group of compounds (XXIB) to give the iodinated compounds (XXIIB);
- step f": stands for the arylation reaction with the suitable pyrazole derivative by a metal catalysed coupling reaction (for example a Buchwald reaction) procedure to give the final compounds (1).

Compounds of formula (IIIB), (VB), (XVIIB) and (XXIIIB) are known compounds or may be prepared according to known methods in the literature.

Compounds of formula (I) may be prepared according to the previous Schemes 1, 2 and 3, once prepared the heterocyclic reactive residue according to known methods to the skilled in the art.

Those skilled in the art will appreciate that in the preparation of the compound of the invention or a solvate thereof it may be necessary and/or desirable to protect one or more sensitive groups in the molecule to prevent undesirable side reactions. Suitable protecting groups for use according to the present invention are well known to those skilled in the art and may be used in a conventional manner. See, for example, "Protective groups in organic synthesis" by T.W. Greene and P.G.M. Wuts (John Wiley & sons 1991) or "Protecting Groups" by P.J. Kocienski (Georg Thieme Verlag 1994). Examples of suitable amino protecting groups include acyl type protecting groups (e.g. formyl, trifluoroacetyl, acetyl), aromatic urethane type protecting groups (e.g. benzyloxycarbonyl (Cbz) and substituted Cbz), aliphatic urethane protecting groups (e.g. 9-fluorenylmethoxycarbonyl (Fmoc), t-butyloxycarbonyl (Boc), isopropyloxycarbonyl, cyclohexyloxycarbonyl) and alkyl type protecting groups (e.g. benzyl, trityl, chlorotrityl). Examples of suitable oxygen protecting groups may include for example alky silyl groups, such as trimethylsilyl or tert-butyldimethylsilyl; alkyl ethers such as tetrahydropyranyl or tert-butyl; or esters such as acetate
Pharmaceutical acceptable salts may also be prepared from other salts, including other pharmaceutically acceptable salts, of the compound of formula (I) using conventional methods.

The compounds of formula (I) may readily be isolated in association with solvent molecules by crystallisation or evaporation of an appropriate solvent to give the corresponding solvates.

When a specific enantiomer of a compound of general formula (I) is required, this may be obtained for example by resolution of a corresponding enantiomeric mixture of a compound of formula (I) using conventional methods. Thus the required enantiomer may be obtained from the racemic compound of formula (I) by use of chiral HPLC procedure.

The subject invention also includes isotopically-labelled compounds, which are identical to those recited in formula (I) and following, but for the fact that one or more atoms are replaced by an atom having an atomic mass or mass number different from the atomic mass or mass number usually found in nature. Examples of isotopes that can be incorporated into compounds of the invention and pharmaceutically acceptable salts thereof include isotopes of hydrogen, carbon, nitrogen, oxygen, phosphorous, sulphur, fluorine, iodine, and chlorine, such as ²H, ³H, ¹¹C, ¹³C, ¹⁴C, ¹⁵N, ¹⁷O, ¹⁸O, ³¹P, ³²P, ³⁵S, ¹⁸F, ³⁶Cl, ¹²³l and ¹²⁵l.

Compounds of the present invention and pharmaceutically acceptable salts of said compounds that contain the aforementioned isotopes and/or other isotopes of other atoms are within the scope of the present invention. Isotopically-labelled compounds of the present invention, for example those into which radioactive isotopes such as ³H, ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. ¹¹C and ¹⁸F isotopes are particularly useful in PET (positron emission tomography), and ¹²⁵I isotopes are particularly useful in SPECT (single photon emission computerized tomography), all useful in brain imaging. Further, substitution with heavier isotopes such as deuterium, i.e., ²H, can afford certain therapeutic advantages resulting from greater metabolic stability, for example increased *in vivo* half-life or reduced dosage requirements and, hence, may be preferred in some circumstances. Isotopically labelled compounds of formula I and following of this invention can generally be prepared by carrying out the procedures disclosed in the Schemes and/or in the Examples below, by substituting a readily available isotopically labelled reagent for a non-isotopically labelled reagent.

The CRF receptor antagonists of the present invention demonstrate activity at the CRF receptor site including CRF 1 and CRF 2 receptors and may be used in the treatment of conditions mediated by CRF or CRF receptors.

The effectiveness of a compound as a CRF receptor antagonist may be determined by various assay methods. Suitable CRF antagonists of this invention are capable of inhibiting the specific binding of CRF to its receptor and antagonizing activities associated with CRF. A compound of structure (I) may be assessed for activity as a CRF antagonist by one or more generally accepted assays for this purpose, including (but not limited to) the assays disclosed by DeSouza et al. (J. Neuroscience 7: 88,1987) and Battaglia et al. (Synapse 1: 572,1987).

The CRF receptors-binding assay was performed by using the homogeneous technique of scintillation proximity (SPA). The ligand binds to recombinant membrane preparation expressing the CRF receptors which in turn bind to wheatgerm agglutinin coated SPA beads. In the Experimental Part will be disclosed the details of the experiments.

With reference to CRF receptor binding affinities, CRF receptor antagonists of this invention have a Ki less than 10 µM. In a preferred embodiment of this invention, a CRF receptor antagonist has a Ki comprised in a range from 0.1 µM and 10 µM.
In a more preferred embodiment the value of Ki is less than 0.1 µM. As set forth in greater detail below, the Ki values of representative compounds of this invention were assayed by the methods set forth in Example 3.

Compounds of the invention are useful in the treatment of central nervous system disorders where CRF receptors are involved. In particular in the treatment or prevention of major depressive disorders including bipolar depression, unipolar depression, single or recurrent major depressive episodes with or without psychotic features, catatonic features, melancholic features, atypical features or postpartum onset, the treatment of anxiety and the treatment of panic disorders. The term anxiety includes anxiety disorders, such as panic disorders with or without agoraphobia, agoraphobia, phobias, for example, social phobias or agoraphobia, obsessive-compulsive disorder, stress disorders including post-traumatic stress disorders, generalised anxiety disorders, acute stress disorders and mixed anxiety-depression disorders. Other mood disorders encompassed within the term major depressive disorders include dysthymic disorder with early or late onset and with or without atypical features, neurotic depression, post traumatic stress disorders and social phobia; dementia of the Alzheimer's type, with early or late onset, with depressed mood; vascular dementia with depressed mood; mood disorders induced by alcohol, amphetamines, cocaine, hallucinogens, inhalants, opioids, phencyclidine, sedatives, hypnotics, anxiolytics and other substances; schizoaffective disorder of the depressed type; and adjustment disorder with depressed mood. Major depressive disorders may also result from a general medical condition including, but not limited to, myocardial infarction, diabetes, miscarriage or abortion, etc.

Compounds of the invention are also useful in the treatment or prevention of schizophrenic disorders including paranoid schizophrenia, disorganised schizophrenia, catatonic schizophrenia, undifferentiated schizophrenia, residual schizoprenia.

Compounds of the invention are useful as analgesics. In particular they are useful in the treatment of traumatic pain such as postoperative pain; traumatic avulsion pain such as brachial plexus; chronic pain such as arthritic pain such as occurring in osteo-, rheumatoid or psoriatic arthritis; neuropathic pain such as post-herpetic neuralgia, trigeminal neuralgia, segmental or intercostal neuralgia, fibromyalgia, causalgia, peripheral neuropathy, diabetic neuropathy, chemotherapy-induced neuropathy, AIDS related neuropathy, occipital neuralgia, geniculate neuralgia, glossopharyngeal neuralgia, reflex sympathetic dystrophy, phantom limb pain; various forms of headache such as migraine, acute or chronic tension headache, temporomandibular pain, maxillary sinus pain, cluster headache; odontalgia; cancer pain; pain of visceral origin; gastrointestinal pain; nerve entrapment pain; sport's injury pain; dysmennorrhoea; menstrual pain; meningitis; arachnoiditis; musculoskeletal pain; low back pain e.g. spinal stenosis; prolapsed disc; sciatica; angina; ankylosing spondyolitis; gout; burns; scar pain; itch; and thalamic pain such as post stroke thalamic pain.

Compounds of the invention are also useful for the treatment of dysfunction of appetite and food intake and in circumstances such as anorexia, anorexia nervosa and bulimia.

Compounds of the invention are also useful in the treatment of sleep disorders including dysomnia, insomnia, sleep apnea, narcolepsy, and circadian ritmic disorders.

Compounds of the invention are also useful in the treatment or prevention of cognitive disorders. Cognitive disorders include dementia, amnestic disorders and cognitive disorders not otherwise specified.

Furthermore compounds of the invention are also useful as memory and/or cognition enhancers in healthy humans with no cognitive and/or memory deficit.

Compounds of the invention are also useful in the treatment of tolerance to and dependence on a number of substances. For example, they are useful in the treatment of dependence on nicotine, alcohol, caffeine, phencyclidine (phencyclidine like compounds), or in the treatment of tolerance to and dependence on opiates (e.g. cannabis, heroin, morphine) or benzodiazepines; in the treatment of cocaine, sedative ipnotic, amphetamine or amphetamine- related drugs (e.g. dextroamphetamine, methylamphetamine) addiction or a combination thereof.

Compounds of the invention are also useful as anti-inflammatory agents. In particular they are useful in the treatment of inflammation in asthma, influenza, chronic bronchitis and rheumatoid arthritis; in the treatment of inflammatory diseases of the gastrointestinal tract such as Crohn's disease, ulcerative colitis, postoperative gastric ileus (POI), inflammatory bowel disease (IBD) and non-steroidal anti-inflammatory drug induced damage; inflammatory diseases of the skin such as herpes and eczema; inflammatory diseases of the bladder such as cystitis and urge incontinence; and eye and dental inflammation.

Compounds of the invention are also useful in the treatment of allergic disorders, in particular allergic disorders of the skin such as urticaria, and allergic disorders of the airways such as rhinitis.

Compounds of the invention are also useful in the treatment of emesis, i.e. nausea, retching and vomiting. Emesis includes acute emesis, delayed emesis and anticipatory emesis. The compounds of the invention are useful in the treatment of emesis however induced. For example, emesis may be induced by drugs such as cancer chemotherapeutic agents such as alkylating agents, e.g. cyclophosphamide, carmustine, lomustine and chlorambucil; cytotoxic antibiotics, e.g. dactinomycin, doxorubicin, mitomycin-C and bleomycin; antimetabolites, e.g. cytarabine, methotrexate and 5- fluorouracil; vinca alkaloids, e.g. etoposide, vinblastine and vincristine; and others such as cisplatin, dacarbazine, procarbazine and hydroxyurea; and combinations thereof; radiation sickness; radiation therapy, e.g. irradiation of the thorax or abdomen, such as in the treatment of cancer; poisons; toxins such as toxins caused by metabolic disorders or by infection, e.g. gastritis, or released during bacterial or viral gastrointestinal infection; pregnancy; vestibular disorders, such as motion sickness, vertigo, dizziness and Meniere's disease; post-operative sickness; gastrointestinal obstruction; reduced gastrointestinal motility; visceral pain, e.g. myocardial infarction or peritonitis; migraine; increased intercranial pressure; decreased intercranial pressure (e.g. altitude sickness); opioid analgesics, such as morphine; and gastro-oesophageal reflux disease, acid indigestion, over-indulgence of food or drink, acid stomach, sour stomach, waterbrash/regurgitation, heartburn, such as episodic heartburn, nocturnal heartburn, and meal-induced heartburn and dyspepsia.

Compounds of the invention are of particular use in the treatment of gastrointestinal disorders such as irritable bowel syndrome (IBS); skin disorders such as psoriasis, pruritis and sunburn; vasospastic diseases such as angina, vascular headache and Reynaud's disease; cerebral ischeamia such as cerebral vasospasm following subarachnoid haemorrhage; fibrosing and collagen diseases such as scleroderma and eosinophilic fascioliasis; disorders related to immune enhancement or suppression such as systemic lupus erythematosus and rheumatic diseases such as fibrositis; and cough.

Compounds of the invention are useful for the treatment of neurotoxic injury which follows cerebral stroke, thromboembolic stroke, hemorrhagic stroke, cerebral ischemia, cerebral vasospam, hypoglycemia, hypoxia, anoxia, perinatal asphyxia cardiac arrest.

The invention therefore provides a compound of formula (I) or a pharmaceutically acceptable salt or solvate thereof for use in therapy, in particular in human medicine.

There is also provided as a further aspect of the invention the use of a compound of formula (I) or a pharmaceutically acceptable salt or solvate thereof in the preparation of a medicament for use in the treatment of conditions mediated by CRF.

In an alternative or further aspect there is provided a method for the treatment of a mammal, including man, in particular in the treatment of condition mediated by CRF, comprising administration of an effective amount of a compound of formula (I) or a pharmaceutically acceptable salt or a solvate thereof.

While it is possible that, for use in therapy, a compound of the present invention may be administered as the raw chemical, it is preferable to present the active ingredient as a pharmaceutical formulation e. g. when the agent is in admixture with a suitable pharmaceutical excipient, diluent or carrier selected with regard to the intended route of administration and standard pharmaceutical practice.

In a further aspect, the invention provides a pharmaceutical composition comprising at least one compound of the invention or a pharmaceutically acceptable derivative thereof in association with a pharmaceutically acceptable carrier and/or excipient. The carrier and/or excipient must be "acceptable" in the sense of being compatible with the other ingredients of the formulation and not deletrious to the receipient thereof.

Accordingly, the present invention further provides a pharmaceutical formulation comprising at least one compound of the invention or a pharmaceutically acceptable derivative thereof, in association with a pharmaceutically acceptable carrier and/or excipient. The carrier and/or excipient must be "acceptable" in the sense of being compatible with the other ingredients of the formulation and not deletrious to the receipient thereof.

There is further provided by the present invention a process of preparing a pharmaceutical composition, which process comprises mixing at least one compound of the invention or a pharmaceutically acceptable derivative thereof, together with a pharmaceutically acceptable carrier and/or excipient.

The pharmaceutical compositions may be for human or animal usage in human and veterinary medicine and will typically comprise any one or more of a pharmaceutically acceptable diluent, carrier or excipient. Acceptable carriers or diluents for therapetic use are well known in the pharmaceutical art, and are described, for example, in Remington's Pharmaceutical Sciences, Mack Publishing Co. (A. R. Gennaro edit. 1985). The choice of pharmaceutical carrier, excipient or diluent can be selected with regard to the intended route of administration and standard pharmaceutical practice. The pharmaceutical compositions may comprise as - or in addition to - the carrier, excipient or diluent any suitable binder(s), lubricant(s), suspending agent(s), coating agent(s), solubilising agent(s).

Preservatives, stabilisers, dyes and even flavouring agents may be provided in the pharmaceutical composition. Examples of preservatives include sodium benzoate, sorbic acid and esters of p-hydroxybenzoic acid. Antioxidants and suspending agents may be also used.

There may be different composition/formulation requirements dependent on the different delivery systems. By way of example, the pharmaceutical composition of the present invention may be formulated to be delivered using a mini-pump or by a mucosal route, for example, as a nasal spray or aerosol for inhalation or ingestable solution, or parenterally in which the composition is formulated by an injectable form, for delivery, by, for example, an intravenous, intramuscular or subcutaneous route. Alternatively, the formulation may be designed to be delivered by both routes.

Where the agent is to be delivered mucosally through the gastrointestinal mucosa, it should be able to remain stable during transit though the gastrointestinal tract; for example, it should be resistant to proteolytic degradation, stable at acid pH and resistant to the detergent effects of bile.

Where appropriate, the pharmaceutical compositions can be administered by inhalation, in the form of a suppository or pessary, topically in the form of a lotion, solution, cream, ointment or dusting powder, by use of a skin patch, orally in the form of tablets containing excipients such as starch or lactose, or in capsules or ovules either alone or in admixture with excipients, or in the form of elixirs, solutions or suspensions containing flavouring or colouring agents, or they can be injected parenterally, for example intravenously, intramuscularly or subcutaneously. For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner.

For some embodiments, the agents of the present invention may also be used in combination with a cyclodextrin. Cyclodextrins are known to form inclusion and non-inclusion complexes with drug molecules. Formation of a drugcyclodextrin complex may modify the solubility, dissolution rate, bioavailability and/or stability property of a drug molecule. Drug-cyclodextrin complexes are generally useful for most dosage forms and administration routes. As an alternative to direct complexation with the drug the cyclodextrin may be used as an auxiliary additive, e. g. as a carrier, diluent or solubiliser. Alpha-, betaand gamma-cyclodextrins are most commonly used and suitable examples are described in WO-A-91/11172, WO-A-94/02518 and WO-A-98/55148.

In a preferred embodiment, the agents of the present invention are delivered systemically (such as orally, buccally, sublingually), more preferably orally.

Hence, preferably the agent is in a form that is suitable for oral delivery.

It is to be understood that not all of the compounds need be administered by the same route. Likewise, if the composition comprises more than one active component, then those components may be administered by different routes.

The compounds of the invention may be milled using known milling procedures such as wet milling to obtain a particle size appropriate for tablet formation and for other formulation types. Finely divided (nanoparticulate) preparations of the compounds of the invention may be prepared by processes known in the art, for example see International Patent Application No. WO 02/00196 (SmithKline Beecham).

For oral administration, the pharmaceutical compositions may take the form of, for example, tablets or capsules prepared by conventional means with pharmaceutically acceptable excipients such as binding agents (e.g. pregelatinised maize starch, polyvinylpyrrolidone or hydroxypropyl methylcellulose); fillers (e.g. lactose, microcrystalline cellulose or calcium hydrogen phosphate); lubricants (e.g. magnesium stearate, talc or silica); disintegrants (e.g. potato starch or sodium starch glycollate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g. sorbitol syrup, cellulose derivatives or hydrogenated edible fats); emulsifying agents (e.g. lecithin or acacia); non-aqueous vehicles (e.g. almond oil, oily esters, ethyl alcohol or fractionated vegetable oils); and preservatives (e.g. methyl or propyl-p-hydroxybenzoates or sorbic acid). The preparations may also contain buffer salts, flavouring, colouring and sweetening agents as appropriate.

Preparations for oral administration may be suitably formulated to give controlled release of the active compound.

For buccal administration the composition may take the form of tablets or formulated in conventional manner.

The compounds of the invention may be formulated for parenteral administration by bolus injection or continuous infusion. Formulations for injection may be presented in unit dosage form e.g. in ampoules or in multi-dose containers, with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for constitution with a suitable vehicle, e.g. sterile pyrogen-free water, before use.

The compounds of the invention may be formulated for topical administration in the form of ointments, creams, gels, lotions, pessaries, aerosols or drops (e.g. eye, ear or nose drops). Ointments and creams may, for example, be formulated with an aqueous or oily base with the addition of suitable thickening and/or gelling agents. Ointments for administration to the eye may be manufactured in a sterile manner using sterilised components.

Lotions may be formulated with an aqueous or oily base and will in general also contain one or more emulsifying agents, stabilising agents, dispersing agents, suspending agents, thickening agents, or colouring agents. Drops may be formulated with an aqueous or non-aqueous base also comprising one or more dispersing agents, stabilising agents, solubilising agents or suspending agents. They may also contain a preservative.

The compounds of the invention may also be formulated in rectal compositions such as suppositories or retention enemas, e.g. containing conventional suppository bases such as cocoa butter or other glycerides.

The compounds of the invention may also be formulated as depot preparations. Such long acting formulations may be administered by implantation (for example subcutaneously or intramuscularly) or by intramuscular injection. Thus, for example, the compounds of the invention may be formulated with suitable polymeric or hydrophobic materials (for example as an emulsion in an acceptable oil) or ion exchange resins, or as sparingly soluble derivatives, for example, as a sparingly soluble salt.

For intranasal administration, the compounds of the invention may be formulated as solutions for administration via a suitable metered or unitary dose device or alternatively as a powder mix with a suitable carrier for administration using a suitable delivery device.

A proposed dose of the compounds of the invention is 1 to about 1000mg per day. It will be appreciated that it may be necessary to make routine variations to the dosage, depending on the age and condition of the patient and the precise dosage will be ultimately at the discretion of the attendant physician or veterinarian. The dosage will also depend on the route of administration and the particular compound selected.
Thus for parenteral administration a daily dose will typically be in the range of 1 to about 100 mg, preferably 1 to 80 mg per day. For oral administration a daily dose will typically be within the range 1 to 300 mg e.g. 1 to 100 mg.

### EXAMPLES

in the Intermediates and Examples unless otherwise stated:

Melting points (m.p.) were determined on a Gallenkamp m.p. apparatus and are uncorrected All temperatures refers to °C. Infrared spectra were measured on a FT-IR instrument. Proton Magnetic Resonance (¹H-NMR) spectra were recorded at 400 MHz, chemical shifts are reported in ppm downfield (d) from Me₄Si, used as internal standard, and are assigned as singlets (s), doublets (d), doublets of doublets (dd), triplets (t), quartets (q) or multiplets (m). Column chromathography was carried out over silica gel (Merck AG Darmstaadt, Germany). The following abbreviations are used in text: EtOAc = ethyl acetate, cHex = cyclohexane, CH₂Cl₂ = dichloromethane, Et₂O = dietyl ether, DMF = N,N'-dimethylformamide, NMP = N-methyl pyrrolidinone, DIPEA = N,N-diisopropylethylamine, DME = ethylene glycol dimethyl ether, MeOH = methanol, Et₃N = triethylamine, TFA = trifluoroacetic acid, THF = tetrahydrofuran, DIBAL-H = diisobutylaluminium hydride, DMAP = dimethylaminopyridine, LHMDS = lithium hexamethyldisilazane, DMA = dimethylacetamide, NMM = N-methylmorpholine, DAST = (diethylamino)sulfur trifluoride, Tlc refers to thin layer chromatography on silica plates, and dried refers to a solution dried over anhydrous sodium sulphate; r.t. (RT) refers to room temperature.

### Intermediate 1

### Ethyl 2 4-dichloro-6-methyl-3-pyridinecarboxylate

The title compound was prepared according to an already published procedure: Mittelbach, Martin; *Synthesis,* 1988, 6, p.479-80.

### Intermediate 2

### Ethyl 2-chloro-6-methyl-4-[3-(1,3-thiazol-2-yl)-1H-pyrazol-1-yl]-3-pyridinecarboxylate

To a solution of 2-(1*H*-pyrazol-3-yl)-1,3-thiazole (7.71 g, 1.05 eq) in anh. DMF (61 mL), at 0°C, under N₂, was added NaH 60% in mineral oil (2.03 g, 1.05 eq) and the reaction mixture was stirred for 10 min. at 0°C and then for 1hr at room temperature. Intermediate 1 (11.34 g, 48.0 mmol) was then added as a solution in anh. DMF (35 mL) at 0°C and the resulting solution was heated at 110°C for 3 hr. The reaction was then quenched with water, extracted with EtOAc, washed with brine, dried over anh. Na₂SO₄, filtered and concentrated *in vacuo.* The crude product was purified by flash chromatography (silica gel, cHex/EtOAc 7:3) to give 7.02 g of the title compound as a white solid.
NMR (¹H, CDCl₃): δ 7.91 (d, 1H), 7.91 (d, 1H), 7.41 (d, 1H), 7.31 (s, 1H), 7.18 (d, 1H), 4.50 (q, 2H), 2.78 (s, 3H), 1.25 (t, 3H).
MS *(m*/*z):* 349 [MH]⁺.

### Intermediate 3

### {2-Chloro-6-methyl-4-[3-(1,3-thiazol-2-yl)-1H-pyrazol-1-yl]-3-pyridinyl}methanol

To a solution of intermediate 2 (1.5 g, 4.3 mmol) in anh. CH₂Cl₂ (30 mL), at -78°C, under N₂, was added DIBAI-H 1.0 M in cyclohexane (12.9 mL, 3.0 eq). The reaction mixture was stirred for 1 hr at -78°C and then for 1 hr at room temperature. The reaction was then quenched with a saturated solution of Rochelle's salt, extracted with EtOAc, washed with brine, dried over anh. Na₂SO₄, filtered and concentrated *in vacuo.* The crude product was purified by flash chromatography (silica gel, cHex/EtOAc 1:1) to give 1.02 g of the title compound as a white solid.
NMR (¹H, CDCl₃): δ 8.05 (d, 1H), 7.90 (d, 1H), 7.40 (d, 1H), 7.25 (s, 1H), 7.10 (d, 1H), 4.65 (S, 2H), 4.0 (bs, 1H), 2.60 (s, 3H).
MS *(m*/*z):* 307 [M]⁺.

### Intermediate 4

### 2-chloro-6-methyl-4-[3-(1,3-thiazol-2-yl)-1H-pyrazol-1-yl]-3-pyridinecarbaldehyde

To a solution of intermediate 3 (150 mg, 0.5 mmol) in anh. CH₂Cl₂ (5 mL), at room temperature, under N₂, was added the Dess Martin periodinane (237 mg, 1.12 eq) and the reaction mixture was stirred for 1 hr at room temperature. The reaction was then quenched with a solution of 0.5 g of sodium thiosulfate dissolved in a saturated solution of sodium bicarbonate, extracted with EtOAc, washed with brine, dried over Na₂SO₄, filtered and concentrated *in vacuo.* The crude product was purified by flash chromatography (silica gel, cHex/EtOAc 1:1) to give 124 mg of the title compound as a white solid.
NMR (¹H, CDCl₃): δ 10.4 (s, 1H), 8.0-7.9 (2d, 2H), 7.40 (2d, 2H), 7.10 (s, 1H), 2.70 (s, 3H).
MS (*m*/*z*): 305 [MH]⁺.

### Intermediate 5

### 2-Chloro-6-methyl-3-[(E)-2-(methyloxy)ethenyl]-4-[3-(1,3-thiazol-2-yl)-1H-pyrazol-1-yl]pyridine

To a solution of (methoxymethyl)-triphenylphosphonium chloride (4.24 g, 3 eq) in anh. THF (20 mL), at 0 °C, under N₂, was added n-BuLi 1.6 M in cyclohexane (7.73 ml, 12.37 mmol) and the reaction mixture was brought to room temperature and then stirred for 15 min. A solution of intermediate 4 (1.25 g, 4.1 mmol) in anh. THF (15 mL) was then added and the reaction was stirred at room temperature for 1.5 hr. The reaction was then quenched with water, extracted with EtOAc, washed with brine, dried over anh. Na₂SO₄, filtered and concentrated *in vacuo.* The crude product was purified by flash chromatography (silica gel, cHex/EtOAc 4:1) to give 961 mg of the title compound as a white solid (E:Z = 3:2 mixture, used as such in the next step).
NMR (¹H, CDCl₃) principal E product : δ 7.90 (m, 1H), 7.83 (m, 1H), 7.38 (m, 1H), 7.05 (m, 1H), 7.00 (m, 1H), 6.51 (d, 1H), 5.63 (d, 1H), 3.64 (s, 3H), 2.60 (s, 3H).
MS (*m*/*z*): 333 [MH]⁺.

### Intermediate 6

### {2-chloro-6-methyl-4-[3-(1,3-thiazol-2-yl)-1H-pyrazol-1-yl]-3-pyridinyl}acetaldehyde

To a solution of intermediate 5 (936 mg, 2.8 mmol) in anh. THF (15 mL) was added 6N HCl (21 ml, 45 eq) and the reaction mixture was stirred at room temperature for 15 hr. The reaction was then quenched with sat. aq. NaHCO₃ until pH=7, extracted with EtOAc, washed with brine, dried over anh. Na₂SO₄, filtered and concentrated *in vacuo* to give 893 mg of the title compound as a white solid, which was used in the next step without further purification.
NMR (¹H, CDCl₃): δ 9.80 (s, 1H), 7.90-7.80 (2d, 2H), 7.70 (d, 1H), 7.20 (d, 1H), 7.0 (s, 1H), 4.25 (s, 2H), 2.70 (s, 3H).
MS (*m*/*z*): 319 [MH]⁺.

### Intermediate 7

### 2-{2-chloro-6-methyl-4-[3-(1,3-thiazol-2-yl)-1H-pyrazol-1-yl]-3-pyridinyl}ethanol

To a solution of intermediate 6 (903 mg, 2.84 mmol) in anh. MeOH (10 mL) were added CeCl₃ (700 mg, 1 eq) and NaBH₄ (107 mg, 1 eq) and the reaction mixture was stirred at room temperature for 5 min. The reaction was then quenched with water, extracted with ethyl acetate, washed with brine, dried over anh. Na₂SO₄, filtered and concentrated *in vacuo* to give 848 mg of the title compound as a white solid, which was used in the next step without further purification.
NMR (¹H, CDCl₃): δ 8.00 (m, 2H), 7.50 (d, 1H), 7.20 (m, 2H), 4.25 (t, 2H), 3.20 (t, 2H), 2.70 (s, 3H).
MS (*m*/*z*): 321 [MH]⁺.

### Intermediate 8

### 2-chloro-3-2-{[(1,1-dimethylethyl)(dimethyl)silyl]oxy}ethyl)-6-methyl-4-[3-(1,3-thiazol-2-yl)-1H-pyrazol-1-yl]pyridine

To a solution of intermediate 7 (840 mg, 2.6 mmol) in anh. CH₂Cl₂ (10 mL) were added 2,6-lutidine (0.67 ml, 2.2 eq) and *tert*-butyldimethylsilyl triflate (0.89 ml, 1.5 eq) and the reaction mixture was stirred at room temperature for 15 hr. The reaction was then quenched with an aqueous solution of saturated NH₄Cl, extracted with EtOAc, washed with brine, dried over anh. Na₂SO₄, filtered and concentrated *in vacuo.* The product was purified by flash chromatography (silica gel, cHex/EtOAc 3:2) to give 950 mg of the title compound as a colorless oil.
NMR (¹H, CDCl₃): δ 8.20 (d, 1H), 7.75 (d, 1H), 7.35 (d, 1H), 7.00 (m, 2H), 4.00 (t, 2H), 3.05 (t, 2H), 2.55 (s, 3H), 0.80 (s, 9H), -0.10 (s, 6H).
MS (*m*/*z*): 435 [MH]⁺.

### Intermediate 9

### 3-(2-{[(1,1-dimethylethyl)(dimethyl)silyl]oxy}ethyl)-6-methyl-N-[6-(methyloxy)-2-(trifluoromethyl)-3-pyridinyl]-4-[3-(1,3-thiazol-2-yl)-1H-pyrazol-1-yl]-2-pyridinamine

To a solution of intermediate 8 (68 mg, 0.16 mmol) in anh. DME (2 mL) were added Pd₂(dba)₃ (15 mg, 0.1 eq), 2-(dicyclohexylphosphino)-2'-methylbiphenyl (17 mg, 0.3 eq), K₃PO₄ (90 mg, 3 eq) and intermediate 14 (37 mg, 1.2 eq) and the reaction mixture was submitted to microwave irradiation (150W, 100°C, 60 psi) for two 20 min cycles. The reaction was then quenched with an aqueous solution of saturated NH₄Cl, extracted with EtOAc, washed with brine, dried over anh. Na₂SO₄, filtered and concentrated *in vacuo.* The product was purified by flash chromatography (silica gel, CH₂Cl₂/MeOH 99:1) to give 22 mg of the title compound as a yellow foam.
NMR (¹H, CDCl₃): δ 8.40 (d, 1H), 7.85 (bs, 1H), 7.75 (bs, 1H), 7.31 (s, 1H), 7.03 (s, 1H), 6.90 (d, 1H), 6.63 (s, 1H), 4.16 (t, 2H), 3.94 (s, 3H), 2.76 (t, 2H), 2.36 (s, 3H), 0.77 (s, 9H), 0.04 (s, 6H).

### Intermediate 10

### 3-(2-{[(1,1-Dimethylethyl)(dimethyl)silyl]oxy}ethyl)-6-methyl-N-[2-methyl-6-(methyloxy)-3-pyridinyl]-4-[3-(1,3-thiazol-2-yl)-1H-pyrazol-1-yl]-2-pyridinamine

As in intermediate 9, except that intermediate 19 (2-methyl-6-(methyloxy)-3-pyridinamine) was used instead of intermediate 14 (6-(Methyloxy)-2-(trifluoromethyl)-3-pyridinamine).
MS (*m*/*z*): 537 [MH]⁺.

### Intermediate 11

### N-[2,6-Bis(methyloxy)-3-pyridinyl]-3-(2-{[1,1-dimethylethyl)(dimethyl)silyl]oxy}ethyl)-6-methyl-4-[3-(1,3-thiazol-2-yl)-1H-pyrazol-1-yl]-2-pyridinamine

As in intermediate 9, except that 2,6-bis(methyloxy)-3-pyridinamine was used instead of intermediate 14 (6-(Methyloxy)-2-(trifluoromethyl)-3-pyridinamine).
NMR (¹H,CDCl₃): δ 8.70 (d, 1H); 7.86 (d, 1H); 7.75 (d, 1H); 7.32 (d, 1H); 7.03 (d, 1H); 6.60 (s, 1H); 6.32 (d, 1H); 4.15 (t, 2H); 3.99 (s, 3H); 3.88 (s, 3H); 2.47 (bs,1H); 2.77 (t, 2H); 2.46 (s, 3H); 0.82 (s, 12H).
MS (*m*/*z*): 553 [MH]⁺.

### Intermediate 12

### N⁵-{3-(2-{[(1,1-Dimethylethyl)(dimethyl)silyl]oxy}ethyl)-6-methyl-4-[3-(1,3-thiazol-2yl)-1H-pyrazol-1-yl]-2-pyridinyl)-N²,N²,4-trimethyl-2,5-pyridinediamine

As in intermediate 9, except that intermediate 16 (*N*²,*N*²,4-trimethyl-2,5-pyridinediamine) was used instead of intermediate 14 (6-(Methyloxy)-2-(trifluoromethyl)-3-pyridinamine).
NMR(¹H, DMSO-d₆): δ 8.35 (d, 1H), 8.00 (s, 1H), 7.99 (d, 1H), 7.87 (s, 1H), 7.83 (d, 1H), 7.07 (d, 1H), 6.69 (s, 1H), 6.63 (s, 1H), 3.95 (t, 2H), 3.10 (s, 6H), 2.98 (t, 2H), 2.28 (s, 3H), 2.18 (s, 3H), 0.85 (s, 9H), 0.00 (s, 6H).
MS (*m*/*z*): 550 [MH]⁺.

### Intermediate 13

### 6-(methyloxy)-3-nitro-2-(trifluoromethyl)pyridine

To a solution of 2-chloro-3-nitro-5-methoxypyridine (500 mg, 2.6 mmol) in DMA (5 mL), at r.t., under N₂, were added Cu (1 g, 6 eq) and CF₂Br₂ (500 µL, large excess). The reaction mixture was stirred at 100°C for 8 hr. The brown slurry obtained was extracted with Et₂O (3x50 mL). The combined organic extracts were dried under vacuum and the crude product was purified by flash chromatography (silica gel, CH₂Cl₂ 100%) to give the title compound (316 mg, 53%) as a brown oil.
NMR (¹H, CDCl₃): δ 8.35 (d, 1H), 7.2 (d,1H), 4.3 (s,3H).

### Intermediate 14

### 6-(Methyloxy)-2-(trifluoromethyl)-3-pyridinamine

To a solution of intermediate 13 (98 mg, 0.44 mmol) in MeOH (10 mL), at r.t., was added Pd/C 10% (30 mg 30% w/w) and the resulting suspension subjected to hydrogenation (1 atm) for 2 hr. The palladium was filtered off and the solvent was evaporated under reduced pressure. The crude product was purified by flash chromatography (silica gel, CH₂Cl₂/MeOH 95:5) to give the title compound (37 mg, 44%) as a red oil.
NMR (¹H, CDCl₃): δ 7.10 (d, 1H); 6.56 (d,1H); 3.85 (s,3H); 3.82-3.55 (bs, 2H).

### Intermediate 15

### N,N,4-Trimethyl-5-nitro-2-pyridinamine

To 2-chloro-4-methyl-5-nitropyridine (2.78 g, 16.1 mmol), at r.t., under N₂, was added Me₂NH 2N/THF (55.4 mL). The reaction mixture was heated at 80°C for 2 hr. It was cooled down to r.t. and partitioned between CH₂Cl₂ and water. The phases were separated and the aqueous layer was further extracted with CH₂Cl₂ (3x30 mL). The combined organic extracts were dried over anh. Na₂SO₄, the solids were filtered and the solvent evaporated. The crude title compound was used in the next step without further purification (2.97 g, quantitative yield).
NMR (¹H, CDCl₃): δ 8.99 (s, 1H), 6.24 (s, 1H), 3.2 (s, 6H), 2.62 (s, 3H).
MS (*m*/*z*): 182 [MH]⁺.

### Intermediate 16

### N²,N²,4-Trimethyl-2,5-pyridinediamine

To a suspension of intermediate 15 (2.97 g, 16.31 mmol) in a 1:1 mixture of MeOH/H₂O (100 mL), at r.t., under N₂, were added Fe (3.19 g, 3.5 eq) and NH₄Cl (3.04 g, 3.5 eq). The reaction mixture was heated at 80°C for 1.5 hr. The mixture was filtered and the solid was washed with MeOH. The crude was evaporated to dryness and partitioned between CH₂Cl₂ and water. The phases were separated and the aqueous layer was further extracted with CH₂Cl₂ (3x30 mL). The combined organic extracts were dried over anh. Na₂SO₄, the solids were filtered and the solvent evaporated. The crude product was purified by flash-chromatography (silica gel, EtOAc/MeOH 9:1 → 7:3) to give the title compound as a yellow solid (340 mg, 14%)
NMR (¹H, DMSO-d₆): δ 7.52 (s, 1H), 6.36 (s, 1H), 4.17 (bs, 2H), 2.83 (s, 6H), 2.03 (s, 3H).
MS (*m*/*z*): 152 [MH]⁺.

### Intermediate 17

### 2-Methyl-6-(methyloxy)pyridine

To a solution of 6-methyl-pyridin-2-ol (2.5 g, 0.023 mol) in CH₂Cl₂ (10 mL) at r.t., under N₂, were added Ag₂CO₃ (6 g, 1.5 eq.) and Mel (5,6 mL, 4 eq). The solution was stirred at r.t. for 4 days, then Ag₂CO₃ was filtered and washed with CH₂Cl₂, and the organic layer was evaporated to dryness. The crude product was purified by flash chromatography (silica gel, EtOAc/cHex 2:8) to give the title compound (1.9 mg, 67%) as a white solid.
NMR (¹H, CDCl3): δ 7.4 (t, 1H), 6.6 (d,1H), 6.5 (d,1H), 3.8 (s, 3H), 2.4 (s, 3H).

### Intermediate 18

### 2-Methyl-6-(methyloxy)-3-nitropyridine

To intermediate 17 (1.95 g, 0.015 mol) at r.t., was added HNO₃ 60% (7ml). The mixture became reddish brown with generation of heat and NO₂. Conc. H₂SO₄ (7ml) was then added. The solution was stirred at 80°C overnight. Into the cooled reaction mixture was poured ice water (70ml) and the solution was neutralized with CaCO₃. The aqueous solution was extracted with EtOAc (3x50 mL) and the combined organic extracts were dried over anh. Na₂SO₄. The solids were filtered and the solvent was evaporated to dryness to give the title compound (2.25 g, 86%) as a yellow solid.
NMR (¹H, DMSO): δ 8.3 (d, 1H); 6.8 (d, 1H); 4 (s, 3H), 2.7 (s, 3H).

### Intermediate 19

### 2-Methyl-6-(methyloxy)-3-pyridinamine

To a solution of intermediate 18 (2 g, 0.012 mol) in H₂O/MeOH 1:1 (40 mL), were added NH₄Cl (2.2 g, 3.5 eq.) and Fe powder (2.3 g, 3.5 eq.). The reaction mixture was stirred at 80°C for 16 hr. Fe was then filtered and washed with MeOH. The MeOH was evaporated, H₂O was added and the aqueous solution was extracted with EtOAc (3x50 mL). The combined organic extracts were dried over anh. Na₂SO₄ the solids were filtered and the solvent evaporated to dryness to give the title compound (1.35 g, 81 %) as a brown oil.
NMR (¹H, CDCl₃): δ 6.9 (d, 1H), 6.4 (d, 1H), 3.8 (s, 3H), 2.33 (s, 3H).

### EXAMPLE 1

### Synthesis of representative compounds of structure (I)

### Example 1-1

### 6-Methyl-1-[6-(methyloxy)-2-(trifluoromethyl)-3-pyridinyl]-4-[3-(1,3-thiazol-2-yl)-1H-pyrazol-1-yl]-2,3-dihydro-1H-pyrrolo[2,3-b]pyridine

To a solution of intermediate 21 (10 mg, 0.021 mmol) in anh. DMF (1.5 mL) at r.t., were added Et₃N (12 µL, 4 eq) and MsCl (4 µL, 2 eq). The solution was stirred at 56°C for 4 hr. CH₂Cl₂ (5 mL) was added and the reaction quenched with H₂O (3 mL). The aqueous phase was extracted with CH₂Cl₂ (3x2 mL) and the combined organic extracts dried over anh.

Na₂SO₄. The solids were filtered and the solvent evaporated to dryness. The crude product was purified by flash chromatography (silica gel, CH₂Cl₂/MeOH 99:1) to give the title compound (2 mg, 21 %) as a white foam.

### Example 1-2

### 6-Methyl-1-[2-methyl-6-(methyloxy)-3-pyridinyl]-4-[3-(1,3-thiazol-2-yl)-1H-pyrazol-1-yl]-2,3-dihydro-1H-pyrrolo[2,3-b]pyridine

To a solution of intermediate 22 (126 mg, 0.030 mmol) in anh. CH₂Cl₂ (10 mL) at r.t., under N₂, were added Et₃N (0.0167 mL, 4eq), PPh₃ (310 mg, 4eq) and l₂ (305 mg, 4eq). The reaction mixture was stirred at r.t. for 2 hr. Water (10 mL) was then added and the solution extracted with EtOAc (15 mL). The organic layer was dried over anh. Na₂SO₄, the solids were filtered and the solvent evaporated *in vacuo.* The crude compound thus obtained was purified by flash chromatography (silica gel, cHex/EtOAc 6:4) to give 5.9 mg of the title compound as a white solid.

### Example 1-3

### 1-[2,6-Bis(methyloxy)-3-pyridinyl]-6-methyl-4-[3-(1,3-thiazol-2-yl)-1H-pyrazol-1-yl]-2,3-dihydro-1H-pyrrolo[2,3-b]pyridine

To a solution of intermediate 23 (46mg, 0.105 mmol) in anh. CH₂Cl₂ (2 mL) at r.t., under N₂, were added Et₃N (73 µL, 5eq), polymer bound PPh₃ (174mg, 5eq) and CBr₄ (174mg, 5eq). The reaction mixture was stirred at r.t. for 2 hr. Water (10 mL) was then added and the solution extracted with EtOAc (15 mL). The organic layer was dried over anh. Na₂SO₄, the solids were filtered and the solvent evaporated *in vacuo*. The crude compound thus obtained was purified by flash chromatography (silica gel, cHex/EtOAc 7:3) to give 14 mg of the title compound as a white solid.

### Example 1-4

### N,N,4-Trimethyl-5-{6-methyl-4-[3-(1,3-thiazol-2-yl)-1H-pyrazol-1-yl]-2,3-dihydro-1H-pyrrolo[2,3-b]pyridin-1-yl}-2-pyridinamine

To a solution of intermediate 24 (45 mg, 0.1 mmol) in anh. CH₂Cl₂ (2 mL), at r.t., under N₂, were added l₂ (50.8 mg, 2 eq), PPh₃ (52.4 mg, 2 eq) and Et₃N (27 µL, 2 eq). The reaction mixture was stirred at r.t. for 2.5 hr and evaporated to dryness. The crude product was purified by flash chromatography (silica gel, EtOAc/MeOH 95:5 → 7:3 EtOAc/NH₃ (0.5 M in MeOH)) to give the title compound as a yellow solid (3 mg, 10 %).

All the analytical data are set forth in the following Table 1-1.

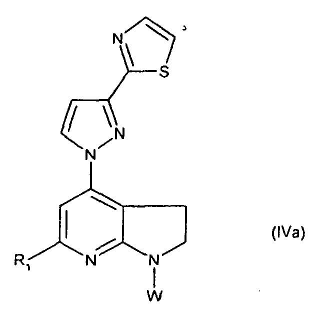

| **Cpd. No.** | **W** | **R₁** | **Analytical Data** |
|---|---|---|---|
| 1-1 | 2-trifluoromethyl-4-methoxypyridin-3-yl | CH₃ | NMR (¹H, CDCl₃): δ 7.9 (d, 1H); 7.8 (d, 1H); 7.6 (d, 1H); 7.3 (d, 1H); 7.0 (d, 1H); 6.9 (d, 1H); 6.7 (s, 1H): 3.9 (s, 3H); 3.8 (t, 2H); 3.5 (t, 2H); 2.33 (s, 3H). |
| | | | MS (*m*/*z*): 459 [MH]⁺. |
| 1-2 | 2-methyl-4-methoxypyridin-3-yl | CH₃ | NMR (¹H, CDCl₃): δ 7.98 (d, 1H), 7.88 (d, 1H), 6.98 (d, 1H), 6.83 (d, 1H), 7.03 (d, 1H), 6.62 (d, 1H), 6.60 (s, 1H), 3.92 (s, 3H), 3.90 (d, 2H), 3.56 (d, 2H), 2.39 (s, 3H), 2.35 (s, 3H). |
| | | | MS (*m*/*z*): 405 [MH]⁺. |
| 1-3 | 2,4-dimethoxypyridin-3-yl | CH₃ | NMR (¹H, CDCl₃): δ 7.98 (d, 1H); 7.87 (d, 1H); 7.72 (d, 1H); 7.34 (d, 1H); 7.06 (d, 1H); 6.67 (s, 1H); 6.36 (d, 1H); 3.96 (t, 2H); 3.96 (s, 3H); 3.94 (s, 3H); 3.51 (t, 2H); 2.37 (s, 3H)δ. |
| | | | MS (*m*/*z*): 421 [MH]⁺. |
| 1-4 | 6-methyl-4-dimethylamino-pyridin-3-yl | CH₃ | NMR (¹H, DMSO-d₆): δ 8.57 (d, 1H), 7.95 (s, 1H), 7.92 (d, 1H), 7.78 (d, 1H), 7.04 (d, 1H), 6.86 (s, 1H), 6.55 (s, 1H), 3.88 (t, 2H), 3.49 (t, 2H), 3.16 (s, 3H), 3.14 (s, 3H), 3.01 (s, 6H). IR (, cm⁻¹): |
| | | | MS (*m*/*z*): 418 [MH]⁺. |

### EXAMPLE 2

### CRF Binding Activity

CRF binding affinity has been determined in vitro by the compounds' ability to displace ¹²⁵I-oCRF and ¹²⁵I-Sauvagine for CRF1 and CRF2 SPA, respectively, from recombinant human CRF receptors expressed in Chinese Hamster Ovary (CHO) cell membranes. For membrane preparation, CHO cells from confluent T-flasks were collected in SPA buffer (HEPES/KOH 50mM, EDTA 2mM; MgCl₂ 10mM, pH 7.4.) in 50mL centrifuge tubes, homogenized with a Polytron and centrifuged (50'000g for 5min at 4°C: Beckman centrifuge with JA20 rotor). The pellet was resuspended, homogenized and centrifuged as before.
The SPA experiment has been carried out in Optiplate by the addition of 100 µL the reagent mixture to 1µL of compound dilution (100% DMSO solution) per well. The assay mixture was prepared by mixing SPA buffer, WGA SPA beads (2.5 mg/mL), BSA (1 mg/mL) and membranes (50 and 5 µg of protein/mL for CRF1 and CRF2 respectively) and 50 pM of radioligand.
The plate was incubated overnight (>18 hrs) at room temperature and read with the Packard Topcount with a WGA-SPA ¹²⁵I counting protocol.

### EXAMPLE 3

### CRF functional assay

Compounds of the invention were characterised in a functional assay for the determination of their inhibitory effect. Human CRF-CHO cells were stimulated with CRF and the receptor activation was evaluated by measuring the accumulation of cAMP.
CHO cells from a confluent T-flask were resuspended with culture medium without G418 and dispensed in a 96-well plate, 25'000c/well, 100 µL/well and incubated overnight. After the incubation the medium was replaced with 100 µL of cAMP IBMX buffer warmed at 37°C (5mM KCI, 5mM NaHCO₃, 154mM NaCl, 5mM HEPES, 2.3mM CaCl₂, 1mM MgCl₂; 1g/L glucose, pH 7.4 additioned by 1 mg/mL BSA and 1mM IBMX) and 1µL of antagonist dilution in neat DMSO. After 10 additional minutes of incubation at 37°C in a plate incubator without CO2, 1µL of agonist dilution in neat DMSO was added. As before, the plate was incubated for 10 minutes and then cAMP cellular content was measured by using the Amersham RPA 538 kit.

All publications, including but not limited to patents and patent applications, cited in this specification are herein incorporated by reference as if each individual publication were specifically and individually indicated to be incorporated by reference herein as though fully set forth.

It is to be understood that the present invention covers all combinations of particular and preferred groups described herein above.

The application of which this description and claims forms part may be used as a basis for priority in respect of any subsequent application. The claims of such subsequent application may be directed to any feature or combination of features described herein. They may take the form of product, composition, process, or use claims and may include, by way of example and without limitation, the following claims:

## Claims

1. Compounds of formula (I) including stereoisomers, prodrugs and pharmaceutically acceptable salts or solvates thereof 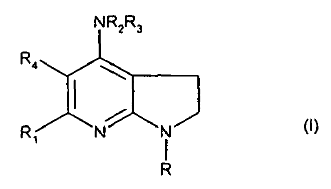 in which:
R is pyridine, which may be substituted by 1 to 4 Z groups;
R₁ is hydrogen, C1-C6 alkyl, C2-C6 alkenyl, C2-C6 alkynyl, halo C1-C6 alkyl, halo C1-C6 alkoxy, halogen, NR₆R₇ or cyano;
R₂ and R₃ together with N form: 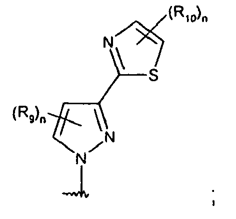
R₄ is hydrogen;
R₅ is a C1-C4 alkyl, -OR₆ or-NR₆R₇;
R₆ is hydrogen or C1-C6 alkyl;
R₇ is hydrogen or C1-C6 alkyl;
R₈ is thiazolyl substituted by 1 to 3 R₉ groups;
R₉ is hydrogen, C3-C7 cycloalkyl, C3-C7 cycloalkenyl, C1-C6 alkyl, C1-C6 alkoxy, halo C1-C6 alkyl, halo C1-C6 alkoxy, hydroxy, halogen, nitro, cyano, -C(O)R₅, C(O)NR₆R₇, phenyl which may be substituted by 1 to 4 R₁₀ groups;
R₁₀ is C1-C6 alkyl, halo C1-C2 alkyl, halogen, nitro, hydroxy, halo C1-C6 alkoxy, C1-C6 alkoxy,or cyano;
Z is selected in a group consisting from halogen, C1-C6 alkyl, C1-C6 alkoxy, halo C1-C6 alkyl, C2-C6 alkenyl, C2-C6 alkynyl, halo C1-C6 alkoxy, -C(O)R₅, -NR₆R₇, cyano, and a group R₈;
n is an integer from 1 to 2.

2. Compounds of formula (lVa), according to claim 1, 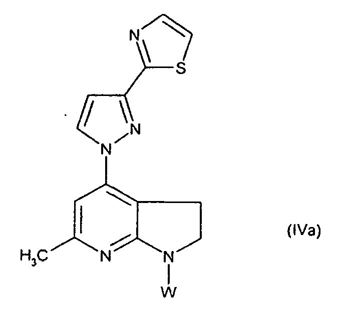 in which W is defined as R in claim 1.

3. Compounds according to the claims from 1 to 2 selected in the group consisting from:
6-methyl-1-[6-(methyloxy)-2-(trifluoromethyl)-3-pyridinyl]-4-[3-(1,3-thiazol-2-yl)-1*H-*pyrazol-1-yl]-2,3-dihydro-1*H*-pyrrolo[2,3-*b*]pyridine;
6-methyl-1-[2-methyl-6-(methyloxy)-3-pyridinyl]-4-[3-(1,3-thiazol-2-yl)-1*H*-pyrazol-1-yl]-2,3-dihydro-1*H*-pyrrolo[2,3-*b*]pyridine;
1-[2,6-bis(methyloxy)-3-pyridinyl]-6-methyl-4-[3-(1,3-thiazol-2-yl)-1*H*-pyrazol-1-yl]-2,3-dihydro-1*H*-pyrrolo[2,3-*b*]pyridine;
*N,N*,4-trimethyl-5-{6-methyl-4-[3-(1,3-thiazol-2-yl)-1*H*-pyrazol-1-yl]-2,3-dihydro-1*H-*pyrrolo[2,3-*b*]pyridin-1-yl}-2-pyridinamine.

4. A process for the preparation of a compound of formula (I) as claimed in claim 1, which comprises the following steps: 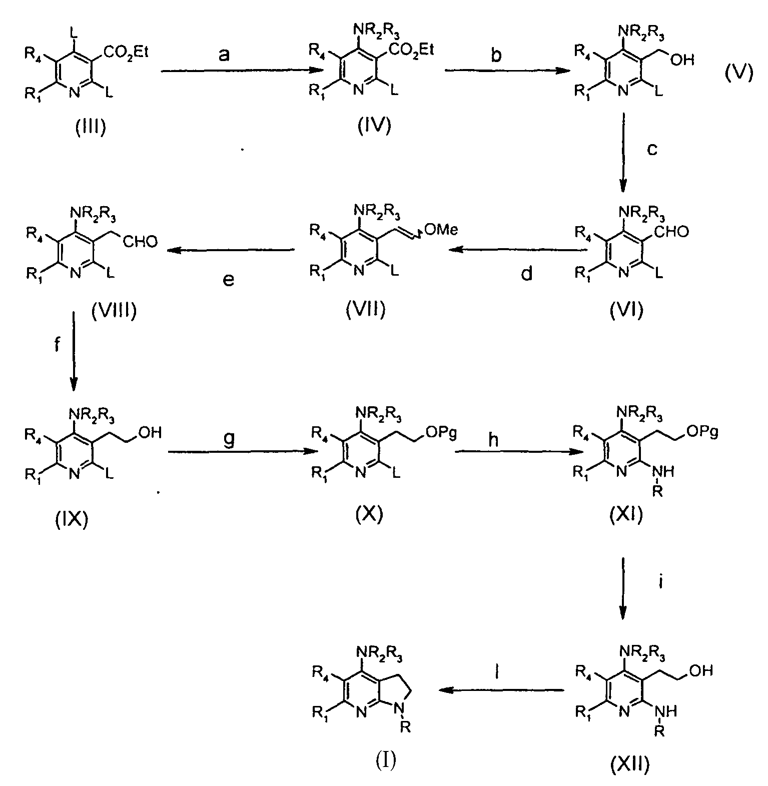 in which
step a stands for conversion of the leaving group L, selected in a group consisting from: halogen or reactive residue of sulphonic acid (e.g. mesylate, tosylate), preferably chloride, in the amino group of compounds (IV), by reaction with the suitable amine NR₂R₃ in basic conditions;
step b stands for reduction of the ester group with a suitable reducing agent (such as DIBAI-H) to hydroxy group of compounds (V);
step c stands for oxidation of the hydroxy group with a suitable oxidising agent (such as Dess-Martin periodinane) to aldehyde group of compound (VI);
step d stands for formation of the aldehyde group of compounds (VIII) by Wittig reaction in the usual conditions, through formation of enol ether followed by acid hydrolysis (step e);
step f stands for reduction of the aldehyde group with a suitable reducing agent (such as NaBH₄) to hydroxy group of compounds (IX);
step g stands for conversion of the hydroxy group in the suitable protecting group of compounds (X)(such as TBS: tert-butyldimethylsilyl);
step h stands for Buchwald reaction by coupling with the suitable amine RNH₂;
step i stands for deprotection reaction to give the hydroxy group of compounds (XII);
step l stands for intramolecular cyclisation by heating after conversion of the hydroxy group of compounds (XII) in a suitable leaving group (such as bromide, by reaction with CBr₄ and PPh₃) to give the final compounds (I).

5. The use of a compound according to anyone of claims from 1 to 3, in the preparation of a medicament for use in the treatment of conditions mediated by CRF (corticotropin-releasing factor).

6. The use of a compound according to anyone of claims from 1 to 3, in the preparation of a medicament for use in the treatment of depression and anxiety.

7. The use of a compound according to anyone of claims from 1 to 3, in the preparation of a medicament for use in the treatment of IBS (irritable bowel disease) and IBD (inflammatory bowel disease).

8. A compound according to anyone of claims from 1 to 3, for use in the treatment of conditions mediated by CRF (corticotropin-releasing factor).

9. A pharmaceutical composition comprising a compound of claims from 1 to 3, in admixture with one or more physiologically acceptable carriers or excipients.

## Patentansprüche

1. Verbindungen der Formel (I), einschließlich Stereoisomere, Prodrugs und pharmazeutisch verträgliche Salze oder Solvate davon 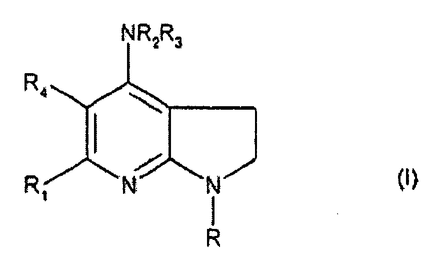 in welchen
R Pyridin, welches mit 1 bis 4 Resten Z substituiert sein kann, ist;
R₁ Wasserstoff, C1-C6-Alkyl, C2-C6-Allcenyl, C2-C6-Alkinyl, Halogen-C1-C6-alkyl, Halogen-C1-C6-alkoxy, Halogen, NR₆R₇ oder Cyano ist;
R₂ und R₃ zusammen mit N 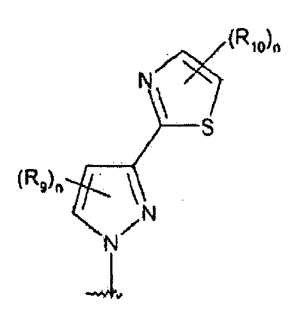 bilden;
R₄ Wasserstoff ist;
R₅ ein C1-C4-Alkyl, -OR₆ oder -NR₆R₇ ist;
R₆ Wasserstoff oder C1-C6-Alkyl ist;
R₇ Wasserstoff oder C1-C6-Alkyl ist;
R₈ Thiazolyl, substituiert mit 1 bis 3 Resten R₉, ist;
R₉ Wasserstoff, C3-C7-Cycloalkyl, C3-C7-Cycloalkenyl, C1-C6-Alkyl, C1-C6-Alkoxy, Halogen-C1-C6-alkyl, Halogen-C1-C6-alkoxy, Hydroxy, Halogen, Nitro, Cyano, -C(O)R₅; C(O)NR₆R₇, Phenyl, welches mit 1 bis 4 Resten R₁₀ substituiert sein kann, ist;
R₁₀ C1-C6-Alkyl, Halogen-C1-C2-alkyl, Halogen, Nitro, Hydroxy, Halogen-C1-C6-alkoxy, C1-C6-Alkoxy oder Cyano ist;
Z ausgewählt ist aus Halogen, C1-C6-Alkyl, C1-C6-Alkoxy, Halogen-C1-C6-alkyl, ,C2-C6-Alkenyl, C2-C6-Alkinyl, Halogen-C1-C6-alkoxy, -C(O)R₅, -NR₆R₇, Cyano und einem Rest R₈;
n eine ganze Zahl von 1 bis 2 ist.

2. Verbindungen der Formel (IVa) gemäß Anspruch 1, 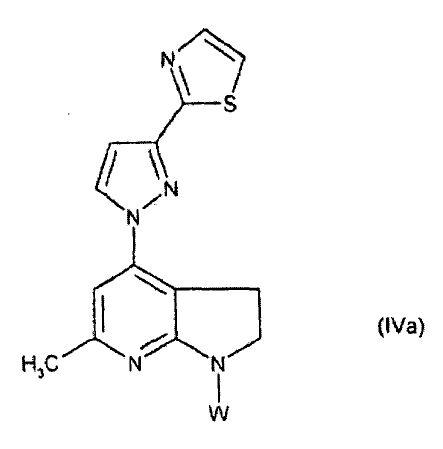 in welchen W wie R in Anspruch 1 definiert ist.

3. Verbindungen gemäß den Ansprüchen 1 bis 2, ausgewählt aus:
6-Methyl-1-[6-(methyloxy)-2-(trifluormethyl)-3-pyridinyl]-4-[3-(1,3-thiazol-2-yl)-1*H-*pyrazol-1-yl]-2,3-dihydro-1*H* pyrrolo[2,3-*b*]pyridin;
6-Methyl-1-[2-methyl-6-(rnethyloxy)-3-pyridinyl]-4-[3-(1,3-thiazol-2-yl)-1*H* pyrazol-1-yl]-2,3-dihydro-1*H*-pyrrolo[2,3-*b*]pyridin;
1-[2,6-Bis(methyloxy)-3-pyridinyl]-6-methyl-4-[3-(1,3-thiazol-2-yl)-1*H*-pyrazol-1-yl]-2,3-dihydro-1*H*-pyrrolo[2,3-*b*]pyridin;
N,N-4-Trimethyl-5-{6-methyl-4-[3-(1,3-thiazol-2-yl)-1*H*-pyrazol-1-yl]-2,3-dihydro-1*H-*pyrrolo[2,3-*b*]pyridin-1-yl-2-pyridinamin.

4. Verfahren zur Herstellung einer Verbindung der Formel (I) gemäß Anspruch 1, welches die folgenden Schritte umfasst: 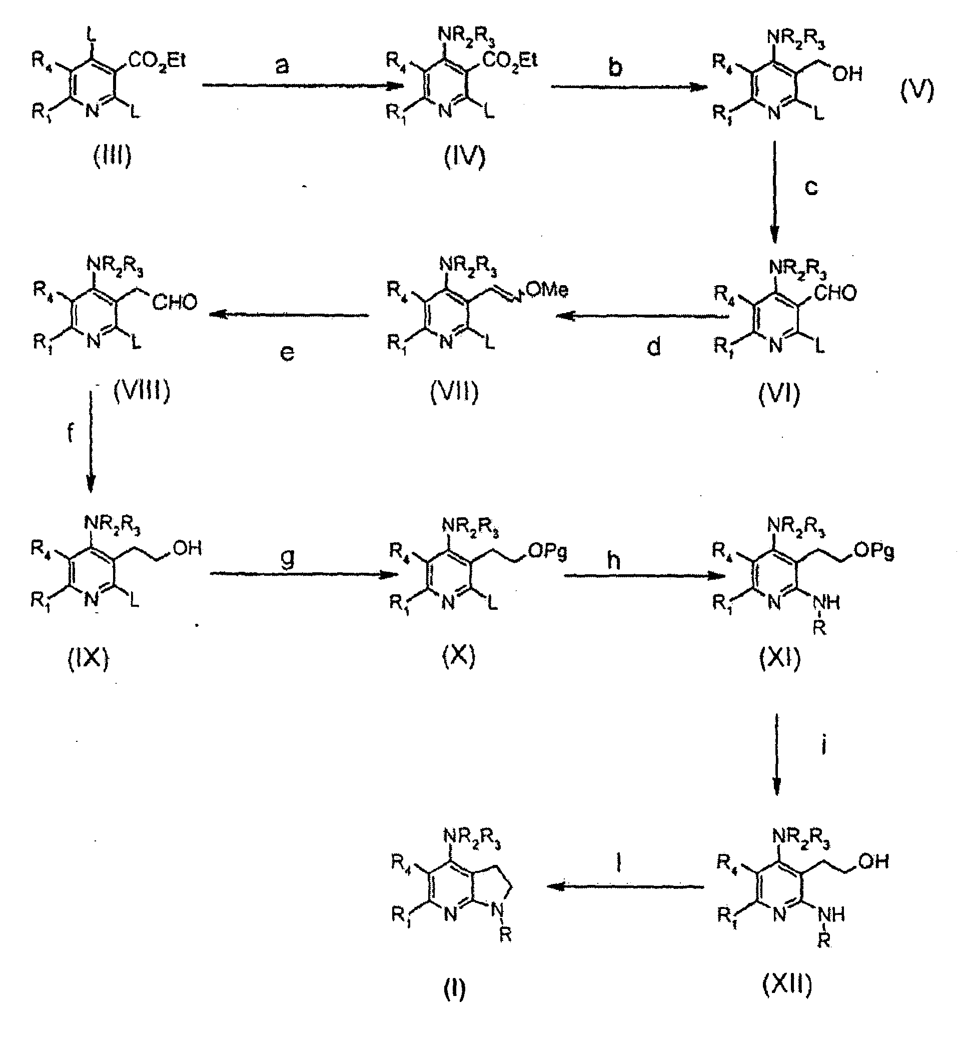 in welchem
Schritt a für die Umwandlung der Abgangsgruppe L, ausgewählt aus Halogen oder einem reaktiven Rest der Sulfonsäure (z.B. Mesylat, Tosylat), vorzugsweise Chlorid, in die Aminogruppe der Verbindungen (IV) durch Reaktion mit dem geeigneten Amin NR₂R₃ in basischen Bedingungen steht;
Schritt b für die Reduktion des Esterrests mit einem geeigneten Reduktionsmittel (wie DIBAI-H) in eine Hydroxygruppe der Verbindungen (V) steht;
Schritt c für die Oxidation der Hydroxygruppe mit einem geeigneten Oxidationsmittel (wie Dess-Martin-Periodinan) in eine Aldehydgruppe der Verbindung (VI) steht;
Schritt d für die Bildung der Aldehydgruppe der Verbindungen (VIII) durch eine Wittig-Reaktion unter den üblichen Bedingungen durch Bildung eines Enolethers, gefolgt von Säurehydrolyse (Schritt e), steht;
Schritt f für die Reduktion der Aldehydgruppe mit einem geeigneten Reduktionsmittel (wie NaBH₄) in eine Hydroxygruppe der Verbindungen (IX) steht;
Schritt g für die Umwandlung der Hydroxygruppe in die geeignete Schutzgruppe der Verbindungen (X) (wie TBS: tert.-Butyldimethylsilyl) steht;
Schritt h für die Buchwaldreaktion durch Kuppeln mit dem geeigneten Amin RNH₂ steht;
Schritt i für eine Entschützungsreaktion steht, um die Hydroxygruppe der Verbindungen (XII) zu ergeben;
Schritt l für eine intramolekulare Cyclisation durch Erwärmung nach Umwandlung der Hydroxygruppe der Verbindungen (XII) in eine geeignete Abgangsgruppe (wie Bromid, durch Reaktion mit CBr₄ und PPh₃) steht, um die Endverbindungen (I) zu ergeben.

5. Verwendung einer Verbindung gemäß einem der Ansprüche 1 bis 3 zur Herstellung eines Medikaments zur Verwendung in der Behandlung von durch CRF (corticotropin-releasing factor) vermittelten Zuständen.

6. Verwendung einer Verbindung gemäß einem der Ansprüche 1 bis 3 zur Herstellung eines Medikaments zur Verwendung in der Behandlung von Depression und Angst.

7. Verwendung einer Verbindung gemäß einem der Ansprüche 1 bis 3 zur Herstellung eines Medikaments zur Verwendung in der Behandlung von IBS (irritable bowel disease) und IBD (inflammatory bowel disease).

8. Verbindung gemäß einem der Ansprüche 1 bis 3 zur Verwendung in der Behandlung von durch CRF (corticotropin-releasing factor) vermittelten Zuständen.

9. Arzneimittel, umfassend eine Verbindung gemäß den Ansprüchen 1 bis 3, in Beimischung mit einem oder mehreren physiologisch verträglichen Trägern oder Exzipienten.

## Revendications

1. Composés de formule (I), ainsi que leurs stéréo-isomères, leurs précurseurs médicamenteux et leurs sels ou produits de solvatation pharmaceutiquement acceptables 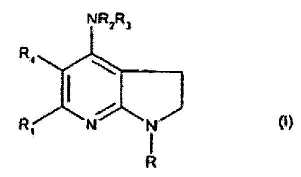 formule dans laquelle :
R représente un groupe pyridine, qui peut être substitué avec 1 à 4 groupes Z ;
R₁ représente un atome d'hydrogène, un groupe alkyle en C₁ à C₆, alcényle en C₂ à C₆, alcynyle en C₂ à C₆, halogénalkyle en C₁ à C₆, halogénalkoxy en C₁ à C₆, halogéno, NR₆R₇ ou cyano ;
R₂ et R₃, conjointement avec N, forment un groupe : 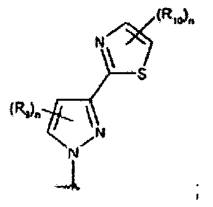
R₄ représente un atome d'hydrogène ;
R₅ représente un groupe alkyle en C₁ à C₄, -OR₆ ou -NR₆R₇ ;
R₆ représente un atome d'hydrogène ou un groupe alkyle en C₁ à C₆ ;
R₇ représente un atome d'hydrogène ou un groupe alkyle en C₁ à C₆ ;
R₈ représente un groupe thiazolyle substitué avec 1 à 3 groupes R₉ ;
R₉ représente un atome d'hydrogène, un groupe cycloalkyle en C₃ à C₇, cycloalcényle en C₃ à C₇, alkyle en C₁ à C₆, alkoxy en C₁ à C₆, halogénalkyle en C₁ à C₆, halogénalkoxy en C₁ à C₆, hydroxy, halogéno, nitro, cyano, -C (O) R₅, C(O)NR₆R₇, phényle qui peut être substitué avec 1 à 4 groupes R₁₀ ;
R₁₀ représente un groupe alkyle en C₁ à C₆, halogénalkyle en C₁ ou C₂, halogéno, nitro, hydroxy, halogénalkoxy en C₁ à C₆, alkoxy en C₁ à C₆ ou cyano ;
Z est choisi dans un groupe consistant en des groupes halogéno, alkyle en C₁ à C₆, alkoxy en C₁ à C₆, halogénalkyle en C₁ à C₆, alcényle en C₂ à C₆, alcynyle en C₂ à C₆, halogénalkoxy en C₁ à C₆, -C (O) R₅, -NR₆R₇, cyano et un groupe R₈ ;
n représente le nombre entier 1 ou 2.

2. Composés de formule (IVa), suivant la revendication 1, 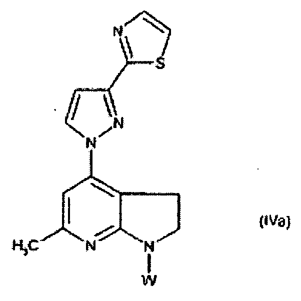 dans laquelle W est défini comme R dans la revendication 1.

3. Composés suivant les revendications 1 et 2, choisis dans le groupe consistant en :
6-méthyl-1-[6-(méthyloxy)-2-(trifluorométhyl)-3-pyridinyl]-4-[3-(1,3-thiazole-2-yl)-1*H*-pyrazole-1-yl]-2,3-dihydro-1*H-*pyrrolo[2,3-*b*]pyridine ;
6-méthyl-1-[2-méthyl-6-(méthyloxy)-3-pyridinyl]-4-[3-(1,3-thiazole-2-yl)-1*H*-pyrazole-1-yl]-2,3-dihydro-1*H*-pyrrolo-[2,3-*b*] pyridine ;
1-[2,6-bis(méthyloxy)-3-pyridinyl]-6-méthyl-4-[3-(1,3-thiazole-2-yl)-1*H*-pyrazole-1-yl]-2,3-dihydro-1*H*-pyrrolo[2,3-*b*]-pyridine ;
*N,N*,4-triméthyl-5-{6-méthyl-4-[3-(1,3-thiazole-2-yl)-1*H*-pyrazole-1-yl]-2,3-dihydro-1*H*-pyrrolo[2,3-*b*]pyridine-1-yl}-2-pyridinamine.

4. Procédé pour la préparation d'un composé de formule (I) suivant la revendication 1, qui comprend les étapes suivantes : 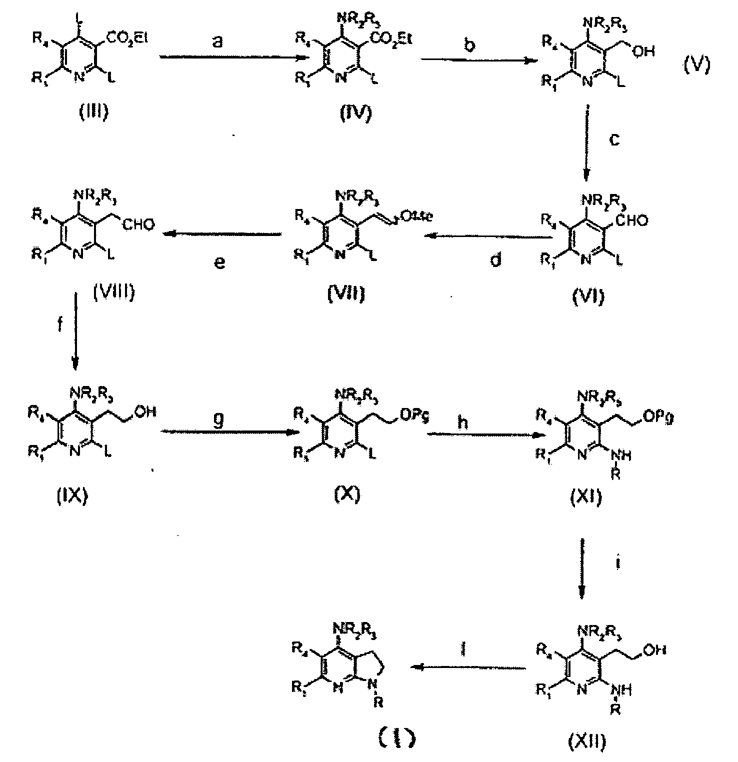 dans lesquelles
l'étape a représente la conversion du groupe partant L, choisi dans un groupe consistant en : un groupe halogéno ou un résidu réactif d'un acide sulfonique (par exemple mésylate, tosylate), de préférence chlorure, dans le groupe amino des composés (IV), par réaction avec une amine NR₂R₃ convenable dans des conditions basiques ;
l'étape b représente la réduction du groupe ester avec un agent réducteur convenable (tel que le DIBA1-H) en un groupe hydroxy des composés (V) ;
l'étape c représente l'oxydation du groupe hydroxy avec un agent oxydant convenable (tel que le périodinane de Dess-Martin) en un groupe aldéhyde du composé (VI) ;
l'étape d représente la formation du groupe aldéhyde des composés (VIII) par une réaction de Wittig dans les conditions habituelles, par formation d'un éther d'énol suivie par une hydrolyse acide (étape e) ;
l'étape f représente la réduction du groupe aldéhyde avec un agent réducteur convenable (tel que NaBH₄) en un groupe hydroxy des composés (IX) ;
l'étape g représente la conversion du groupe hydroxy en le groupe protecteur convenable des composés (X) (par exemple un groupe TBS : tertio-butyldiméthylsilyle) ;
l'étape h représente une réaction de Buchwald par couplage avec l'amine RNH₂ convenable ;
l'étape i représente la réaction de suppression de protection donnant le groupe hydroxy des composés (XII) ;
l'étape l représente une cyclisation intramoléculaire par chauffage après conversion du groupe hydroxy des composés (XII) en un groupe partant convenable (tel qu'un groupe bromure, par réaction avec CBr₄ et PPh₃), ce qui donne les composés (I) finals.

5. Utilisation d'un composé suivant l'une quelconque des revendications 1 à 3, dans la préparation d'un médicament destiné à être utilisé dans le traitement d'affections à médiation par le CRF (facteur de libération de corticotrophine).

6. Utilisation d'un composé suivant l'une quelconque des revendications 1 à 3, dans la préparation d'un médicament destiné à être utilisé dans le traitement de la dépression et de l'anxiété.

7. Utilisation d'un composé suivant l'une quelconque des revendications 1 à 3, dans la préparation d'un médicament destiné à être utilisé dans le traitement de la IBS (maladie du côlon irritable) et de la IBD (maladie intestinale inflammatoire).

8. Composé suivant l'une quelconque des revendications 1 à 3, destiné à être utilisé dans le traitement d'affections à médiation par le CRF (facteur de libération de corticotrophine).

9. Composition pharmaceutique comprenant un composé suivant les revendications 1 à 3, en mélange avec un ou plusieurs supports ou excipients physiologiquement acceptables.
